# EUROPEAN PATENT APPLICATION

(11) **EP 3 327 131 A1**
(43) Date of publication of application: **30.05.2018**
(21) Application number: 16200923.7
(22) Date of filing: 28.11.2016
(51) Int. Cl.: C12P 19/02, C12P 19/14, C12P 7/18, D21C 1/04, D21C 1/06, D21C 3/06, D21C 11/00

(54) **PROCESSING METHOD OF PREPARING A CARBOHYDRATE ENRICHED COMPOSITION**

(71) Applicant: Lenzing AG, 4860 Lenzing (AT)
(72) Inventor: FACKLER, Karin, 1140 Vienna (AT); FEINER, Roland, 4860 Lenzing (AT); MALZNER, Erwin, 4845 Rutzenmoos (AT)
(74) Representative: Grünecker Patent- und Rechtsanwälte PartG mbB

(57) **Abstract**

The present invention relates to methods of processing compositions comprising a lignosulfonate and a carbohydrate, methods of preparing a carbohydrate-enriched composition involving the above processing method, associated carbohydrate-enriched compositions, and uses of the same.

## Description

### Field of the Invention

The present invention relates to a method of processing compositions comprising a lignosulfonate and a carbohydrate. The present invention further relates to a method of preparing a carbohydrate-enriched composition involving the above processing method. The present invention further relates to associated carbohydrate-enriched compositions and uses of the same as a substrate for conversion of the carbohydrate or carbohydrates into a compound chemically distinct from said carbohydrate or carbohydrates. The invention advantageously allows the processing of lignosulfonate- and carbohydrate-comprising compositions, as for instance generated in known wood or biomass pulping processes, to carbohydrate-enriched compositions which are suited for use in further processes for conversion of said carbohydrates into various products of value. The present invention advantageously allows preparatory processes important for downstream conversion of carbohydrates to be integrated into existing processing streams, thus opening the path to obtaining commercially valuable materials from existing carbohydrate byproducts, while obtaining advantageous mass and energy balances of the processing plant.

### Background of the Invention

Spent sulfite liquors (also referred to as sulfite spent liquors or SSL) derived from the sulfite or bisulfite pulping processes, for example Mg-sulfite or Mg-bisulfite pulping processes, are complex aqueous solutions of organic molecules and inorganic salts. The main constituents of such liquors include lignosulfonates; monomeric, dimeric and oligomeric carbohydrate compounds including for instance mannose, xylose, glucose, galactose, arabinose, and rhamnose; and cations derived from the cooking base metal sulfites and metal sulfates, for example Mg(II)-sulfite and Mg(II)-sulfate.

For economic and environmental reasons, modern (bi)sulfite pulp mills (that is, pulp mills based on sulfite or bisulfite processes, for instance Mg-sulfite and Mg-bisulfite processes, for pulping e.g. wood) are usually operated in closed cycles. As part of such cycles, the cooking chemicals (e.g. Mg-sulfite or Mg-bisulfite) are normally recovered from the spent liquors, and the organic molecules are burned to gain thermal and electric energy. As lignosulfonates contain bound sulfur, burning of these compounds is essential for the chemical recovery cycle of a pulp mill; in addition to the energy, burning of e.g. lignosulfonates allows for recovery of valuable sulfur compounds (SO₂) from the combustion gases adsorbed to the MgO ash in the form of Mg-bisulfite, and such recovered sulfur can be recycled for use in further pulping processes. On the other hand, the burning of carbohydrates and other dissolved organic compounds does not contribute to the chemical recovery but only results in energy. In this way, mills employing (bi)sulfite-based pulping processes, for example Mg-(bi)sulfite-based pulping processes, generate excess energy.

Utilizing such carbohydrates as precursors for further conversions, for example conversions effected by appropriately genetically engineered microorganisms, to further products of commercial value would be of economic advantage and would diminish the energy excess of a stand-alone pulp mill. Ideally, it would be advantageous to integrate carbohydrate purification and conversion processes into the material and energy streams of pulp mills, especially pulp mills of the kind described above. Such integration would greatly enhance the value creation of existing (bi)sulfite-based pulping processes, for example Mg-(bi)sulfite-based pulping processes.

The literature describes the utilization of sulfite spent liquor towards obtaining, for example and among other things, ethanol, lactic acid, and single cell protein, e.g. fodder yeast. Industrial conversion of sulfite spent liquors is well known and has been or is being operated worldwide. Such conversion may for example take the form of fermentation, or biotechnological conversion (often inhibited by furaldehydes such as for example furfural, 5-hydroxymethylfurfural (5-HMF) or furfural and 5-HMF; conversion with isolated enzymes, often referred to as "biocatalysts" (often inhibited by phenols); and conversion by chemical catalysis, e.g. by acid-base catalysis or other types of heterogeneous catalysis such as for example metal catalysts (often inhibited by sulfur compounds).

However, integrating such processes, e.g. fermentations directly into the recovery cycle of a (bi)sulfite pulp mill, for example a Mg-(bi)sulfite pulp mill, poses multiple problems. This is especially true when such conversion is to be effected by microbial means, for example using genetically modified organisms (GMOs) or (bio)chemical processes. In some cases, the need for additional sensitive (bio)catalysts or other additional chemicals may force an undesirable deviation of the recovery cycle's ideal operation conditions.

For instance, process chemicals introduced as part of established pulping processes can impede the ability of such engineered microorganisms to effect desired carbohydrate conversions. Conversely, adjustment of existing pulp mill processes to favor a downstream desired microbial conversion can lead to an imbalance of the entire pulp mill. For instance, introduction of nitrogen sources as microbial nutrient supplements into the pulp processing stream can lead to higher NOx emissions, and adjustment of process pH to favor later microbial conversion can shift pH values away from ideal pH values required for efficient pulping. Thus, the conditions prevailing in typical pulping streams are often incompatible with those required for efficient downstream conversion, for example microbial conversion of carbohydrates within this stream. In general, such difficulties have hitherto stood in the way of leveraging the untapped value in existing sulfite or bisulfite pulping processes of the type described above. The types of difficulties which have typically plagued the efficient conversion of carbohydrates contained in the processing streams of existing (bi)sulfite pulping processes are described in more detail below.

One of the primary problems associated with using SSLs as a direct substrate for carbohydrate conversion processes, for instance carbohydrate conversion processes employing microorganisms, relates to the low concentration of carbohydrates in typical SSLs. The main components of SSLs are lignosulfonates; carbohydrates are only minor constituents. Even thickened liquors therefore contain relatively low carbohydrate concentrations (typically between 130 and 200 g/L), compared to other industrially available carbohydrate streams such as molasses and glucose syrups. The high amount of non-carbohydrate compounds and the high dry matter content (i.e. the content of material which, upon complete removal of all water, would remain behind as solids) of approximately 700 - 850 g/L, limits further thickening of sulfite spent thick liquors to gain higher carbohydrate concentrations. Low substrate or educt concentration, respectively the ones of carbohydrates, thus lead to low product concentration, and product isolation and purification costs decrease with increasing product concentration. Higher carbohydrate concentrations than present in SSLs would therefore be needed to ensure the commercial viability of any conversion, especially any microbial conversion, of available carbohydrates into other valuable products. Low substrate, or educt, concentrations, also lead to higher specific volume requirements for conversion reactors and thus higher investment costs (capital expenditures). At the same time, attempts to concentrate, purify or concentrate and purify SSLs by existing membrane separation processes often suffer from severe membrane fouling processes. The result of all of the above is that typical SSLs do not contain carbohydrates in high enough concentrations to allow feasible, or at least profitable, carbohydrate conversion, and efforts to increase the carbohydrate concentration directly out of SSLs entail prohibitively expensive measures which threaten to erode any gain in value achieved.

Another problem impeding the use of SSLs as practical sources of carbohydrate for conversion to products of value relates to the nature of the components typically contained in SSLs. SSLs contain many components likely to exert an undesirable effect on microorganisms or the products produced by them. In some instances, such components in SSLs can be inhibitory or even toxic for microorganisms. For instance, phenolic compounds (lignosulfonates are phenolic compounds), furfural, 5-hydroxymethylfurfural (5-HMF), formic and acetic acid are just some examples of compounds typically present in the spent streams of (bi)sulfite pulping processes, and these components are known to inhibit the activity of microorganisms. In fact, sulfur-containing compounds such as lignosulphonates or sulfur-containing ions are often actually toxic to certain desired conversion processes, in particular certain (bio)catalysts, microorganisms or (bio)catalysts and microorganisms employed in such processes. At the very least, the presence of such compounds threatens to decrease the value of, or even render unmarketable, any final product obtained by such microbial conversion of unprocessed SSLs. For instance, furfural and 5-HMF - both present in typical SSLs - are color precursors which can diminish the final quality of any product obtained by microbial conversion. Additionally, the presence of acids and bases in SSLs (organic acids, metal ions (for example Mg ions) and sulfates) can necessitate a higher consumption of certain process chemicals in order to adjust the pH of the SSL stream into a range required for the desired conversion process, e.g. a microbial conversion process. All of the above factors conspire to complicate, hamper or even preclude conversion of carbohydrates contained in SSLs in the absence of laborious and cost-intensive appropriate prior processing.

From all of the above, it can be taken that state-of-the art conversion processes to produce valuable products in high yields and rates are not directly applicable to SSLs. If the desired conversion, for example microbial conversion, of carbohydrates in typical SSLs is not already rendered commercially unfeasible by issues of low substrate (carbohydrate) concentration, the very components of SSLs themselves stand to preclude such conversion for technical reasons.

Considering all of the above, it would only be economically feasible to integrate carbohydrate conversion processes (as for instance described for conversion of substrates/educts from edible sources (as e.g. in a 1^{st} generation biorefinery) or from other sorts of biomass (as e.g. in a 2^{nd} generation biorefinery)), into a (bi)sulfite pulp mill, for example a Mg-(bi)sulfite pulp mill, if the above obstacles can be overcome.

It has been recognized that monosaccharides derived from non-food biomass have a huge potential as precursors for bio-based chemicals. To gain access to biomass carbohydrates in their monomeric form, several strategies can be employed. In so-called 2^{nd} generation biorefineries the lignocellulosic biomass must be pre-treated before the accessible cell wall polysaccharides can be hydrolysed by chemical or biochemical means (see e.g. Van Walsum, P.: Separation and Purification of Lignocellulose Hydrolyzates. In: Ramasewami, S., Huang, H.-J., Ramarao, B.V. (eds.). Separation and Purification Technologies in Biorefineries, John Wiley & Sons, Chichester, U.K. (2013), 513-533). Most common substrate pre-treatment processes hydrolyze hemicellulose with mineral acids or auto-hydrolysis processes (see e.g. Huang, H.-J. and Ramaswami, S.: Overview of Biomass Conversion Processes and Separation and Purification Technologies. In: Ramasewami, S., Huang, H.-J., Ramarao, B.V. (eds.). Separation and Purification Technologies in Biorefineries, John Wiley & Sons, Chichester, U.K. (2013), 3-36). However alkaline- or solvent-based as well as mechanical or microbial pretreatments have also been proposed. Hemicellulose hydrolysis to monomeric and oligomeric carbohydrates is also an important feature of acidic sulfite pulping in the presence of sulfur dioxide and a cooking base. Such bases can for instance be based on Ca²⁺, Mg²⁺, Na⁺, oxides or hydroxides or NH₄⁺ hydroxide. All of these hydrolysis processes lead not only to monomeric and oligomeric carbohydrates based on e.g. mannose, xylose, glucose, galactose, arabinose, or rhamnose but also to hydrolysis byproducts derived from the hemicelluloses. Most crucial of these byproducts are the carbohydrate dehydration products furfural and 5-HMF, which can be toxic to subsequent biochemical conversion processes, as described above. In addition, organic acids such as acetic acid and formic acid are well known to inhibit fermentations (see e.g. Almeida, J.R.M., Bertilson, M., Gorwa-Grauslund, M.F., Gorsich, S., Lidén, G. Metabolic effects of furaldehydes and impacts on biotechnological processes. Appl. Microbiol. Biotechnol. (2009) 82: 625-638). In addition, as also mentioned above, lignosulfonates from sulfite pulping processes carry phenolic groups which can also inhibit subsequent fermentation and enzymes such as for example oxidoreductases, hydrolases, transferases or any combination thereof, and, due to their sulfur content, may also be toxic to chemical conversion catalysts such as for example catalysts based on Pt, Pd, Ni, Re or any combination thereof.

Separation of inhibitors thus is crucial to be able to operate carbohydrate conversion processes with competitive yields and rates. Several methods have been suggested for detoxification of acid-hydrolyzed biomass such as for instance overliming, using calcium hydroxide, adsorption to active charcoal, solvent extraction, ion exchange, flocculation, enzyme treatments, and nanofiltration. None of these separation steps take into account any process integration into a (bi)sulfite pulp mill, for example a Mg-(bi)sulfite pulp mill with state-of-the-art closed chemical recovery cycles. For instance, overliming and calcium hydroxide introduce cations into the chemical recovery which interfere in bisulfite, e.g. Mg-bisulfite recovery boilers. Adsorbents are insoluble and thus cannot be integrated into liquors destined for burning in conventional thick liquor based (bi)sulfite, e.g. Mg-(bi)sulfite recovery boilers. Basic ion exchange would have to be carried out at a pH that would require introduction of an additional base, which would lead to an undesirable additional load of inorganic products in the liquors. Other methods such as flocculation and solvent extraction also introduce new chemicals into the recovery cycle.

US 5,637,225 and WO 94/26380 disclose a method for separating components of an SSL composition. WO 2009/155982 describes a process for the separation and obligatory crystallization of a xylose-containing, lignosulfonate containing Ca- or Mg-sulfite spent liquor. The product is crystallized xylose. Malmali et al. (Malmali, M., Stickel, J.J., Wickramasinghe, S.R. Sugar concentration and detoxification of clarified biomass by nanofiltration. Separation and Purification Technology (2014), 132, 655-665) describes the separation of impurities from monomeric carbohydrates.

Current state-of-the-art provides no process which would be suitable for processing carbohydrates to further products, e.g. non-carbohydrate products, especially by microbial means, in a manner which is integrable as part of an existing pulping process stream. The present invention addresses these needs.

### Brief description of the Invention

One aspect of the invention provides a method of processing an initial composition comprising a lignosulfonate and a carbohydrate, said method comprising: subjecting the initial composition to a separation procedure whereby an amount of the lignosulfonate is removed from the initial composition, thereby yielding a **l**ignosulfonate-**c**ontaining and **c**arbohydrate-**d**epleted (LCCD) composition and a lignosulfonate-depleted composition; and subjecting the lignosulfonate-depleted composition to a concentration procedure whereby the carbohydrate concentration is increased relative to the carbohydrate concentration in the initial composition, relative to the carbohydrate concentration in the lignosulfonate-depleted composition or relative to the carbohydrate concentration in both the initial composition and the lignosulfonate-depleted composition, thereby yielding a carbohydrate-enriched composition. The carbohydrate concentration may be calculated e.g. based on grams carbohydrate per liter of composition, or based on g/kg dry substance of the composition. In one advantageous embodiment the carbohydrate in the carbohydrate-enriched composition is not crystallized, in particular is not crystallized subsequent to the concentration procedure. In a further embodiment the initial composition is a liquor, for example a (bi)sulfite spent liquor from a biomass, e.g. wood, pulping process. In a further embodiment the carbohydrate-enriched composition is an aqueous carbohydrate-enriched composition, i.e. is aqueous. In a further embodiment, the initial composition is an aqueous initial composition, i.e. is aqueous. In a further embodiment the separation procedure is a chromatographic separation procedure. In a further embodiment the chromatographic separation procedure employs an ion-exchange material, e.g. resin. Preferably, the ion-exchange material, e.g. resin, is a cation-exchange chromatographic material, e.g. resin, preferably a strong acidic ion-exchange material, e.g. resin, preferably a material, e.g. resin wherein the ion-exchanging groups are sulfonic acid moieties. In a further embodiment the chromatographic separation procedure, e.g. the chromatographic separation procedure employing an ion-exchange material, e.g. resin as described above, is performed as a multi-column recycling chromatographic separation procedure such as simulated moving bed chromatography or dual column chromatography.

In one embodiment the concentration procedure employs a semipermeable membrane, an evaporation step, or a semipermeable membrane and an evaporation step. It is especially preferred that the concentration procedure employs a semipermeable membrane, it being understood that other concentration steps may be performed prior to, subsequent to, or both prior and subsequent to the concentration procedure employing a semipermeable membrane. Where the concentration procedure employs a semipermeable membrane, in one embodiment the semipermeable membrane is employed in a diafiltration procedure, a nanofiltration procedure, an ultrafiltration procedure, a reverse osmosis procedure, or any combination thereof. In such procedures, the portion of the composition passing through the membrane is typically referred to as the "permeate", while the portion of the composition which is retained, i.e. does not pass through the membrane, is typically referred to as the "retentate", and these meanings are used herein.

In one embodiment, the molecular weight cutoff of the semipermeable membrane is chosen so as to prevent the passage of at least one carbohydrate through the membrane. This way, the carbohydrate to be concentrated remains in the retentate, while other unwanted substances which can potentially inhibit downstream processing flow through in the permeate. For example, in the event the initial composition comprises additional substances such as furfural, 5-HMF, acetic acid, formic acid, or any combination thereof, such substances may be advantageously removed or their concentration in the retentate decreased by a concentration procedure employing a semipermeable membrane, as such substances permeate through the semipermeable membrane to a higher extent than carbohydrates in the initial composition, in the lignosulfonate-depleted composition or in both the initial composition and the lignosulfonate-depeleted composition, so such substances as furfural, 5-HMF, acetic acid, formic acid, or any combination thereof will typically permeate through the semipermeable membrane in the permeate, while the carbohydrate(s) will remain behind in the retentate.

Of course, if the initial composition comprised furfural, 5-HMF, acetic acid, formic acid, or any combination thereof, then some of these substances, in addition to other substances (e.g. metal ions) may already have been at least partially removed in the initial separation procedure. In such cases, the concentration procedure may function as a concentration/removal procedure, contributing to the removal of any of these substances still present in the lignosulfonate-depleted composition following the separation procedure, to the extent that such substances were not already removed in the preceding separation procedure. In this way, depending on the content of the lignosulfonate-depleted composition and the way in which the concentration procedure is performed, the concentration procedure may simultaneously affect the concentration of carbohydrate to a level above that in the lignosulfonate-depleted composition, above that in the initial composition or above that in both the lignosulfonate-depleted composition and the initial composition, as well as removal/depletion of other non-carbohydrate impurities to a level below that in the lignosulfonate-depleted composition. In such cases, the concentration procedure functions as a concentration/removal procedure. In this way, the conditions of the initial separation procedure and the subsequent concentration procedure can be tuned to one another in concert so that any undesired substances remaining after a pass or passes through the separation procedure can subsequently be removed in one or more steps of the concentration procedure, which in this event functions as a concentration/removal procedure. The result is a highly flexible method which opens access to a carbohydrate-enriched composition which maximizes the aqueous concentration of solubilized carbohydrate, while minimizing the concentration of any substances which threaten to inhibit any subsequent procedures for the conversion of carbohydrate to which the carbohydrate-enriched composition is to be subjected.

Methods for measuring the concentration of the substances mentioned herein, e.g. carbohydrates in general, as well as a specific carbohydrate such as e.g. xylose, glucose, arabinose etc., lignosulfonates as well as other substances such as magnesium ions, calcium ions, sulfates, sulfites, furfural, 5-HMF, acetic acid and formic acid are well known to the skilled person as a set out e.g. in Sumerskii, I., Korntner, P., Zinovyev, G., Rosenau, T., Potthast, A. Fast track for quantitative isolation of lignosulfonates from spent sulfite liquors (2015) RSC Advances, 5 (112), pp. 92732-92742 (for lignosulfonates); NREL Technical Report: NREL/TP-510-42623 (2008): Determination of Sugars, Byproducts and Degradation Products in Liquid Fraction Process Samples (Laboratory Analytical Procedure) (for carbohydrates, acetic acid, furfural, 5-HMF); Tappi Standard method TAPPI T699 om-00: Analysis of pulping liquors by suppressed ion chromatography (for sulfites and sulfates); and European Standard Method: EN ISO 11885 (2009): Wasserbeschaffenheit - Bestimmung von ausgewählten Elementen durch induktiv gekoppelte Plasma-Atom-Enissionsspektrometrie (ICP-OES) (for ions of Mg, Ca and Na). All of these publications are incorporated herein by reference.

In the aspects and embodiments of the invention hereinabove and hereinbelow, it is to be understood that when concentrations of substances are expressed in relative terms, for example the "lignosulfonate-containing and carbohydrate-depleted composition" resulting from subjecting the initial composition to a separation procedure, or the "carbohydrate-enriched composition" resulting from subjecting the lignosulfonate-depleted composition to a concentration procedure, the same measurement method, performed under the same conditions should be used on both starting and final compositions, so that the results obtained relating to the concentration of the substance in question remain comparable.

In a further embodiment of the method of the invention the initial composition additionally comprises (i.e. comprises in addition to a lignosulfonate and a carbohydrate) metal ions such as Mg²⁺, Ca²⁺, Na⁺ or any combination thereof, sulfates, sulfites, furfural, 5-HMF, acetic acid, formic acid, an aldonic acid (for example xylonic acid, mannonic acid or gluconic acid), or any combination thereof. In a further embodiment the separation procedure additionally removes metal ions, sulfates, sulfites, or any combination thereof from the initial composition. In a further embodiment the concentration procedure removes furfural, 5-HMF, acetic acid, formic acid or any combination thereof from the lignosulfonate-depleted composition. In a further embodiment the concentration procedure is performed such that the carbohydrate concentration in the carbohydrate-enriched composition is at least 200g/L at least 300g/L, at least 400g/L, at least 500g/L, at least 600 g/L, at least 700g/L or at least 800g/L. In a further embodiment the carbohydrate may comprise at least one monosaccharide such as xylose; mannose; glucose; galactose; arabinose; rhamnose; at least one disaccharide, oligosaccharide or polysaccharide of any monosaccharide, especially of any of the monosaccharides mentioned above; at least one hemicellulose; at least one hydrolysis product of a hemicellulose; or any combination thereof.

In a further embodiment the initial composition is a composition resulting from pulping or pretreating a lignocellulosic material, such as wood or other biomass, preferably using H₂SO₃, solutions of SO₂ or both.

In a further embodiment the initial composition is a spent sulfite liquor. In a further embodiment the initial composition comprising the lignosulfonate and the carbohydrate is obtained from the stream of a concurrently ongoing procedure. In a further embodiment the concurrently ongoing procedure is a concurrently ongoing pulping procedure, for example an ongoing wood or lignocellulosic biomass pulping procedure. In a further embodiment the method of the invention further comprises the step of returning the LCCD composition to the stream of the concurrently ongoing procedure, in particular to the concurrently ongoing pulping procedure. In a further embodiment, where the concentration procedure employs a semipermeable membrane, an evaporation step or a semipermeable membrane and an evaporation step, the method of the invention further comprises the step of returning the permeate of the concentration procedure employing a semipermeable membrane, the condensate of the concentration procedure employing an evaporation step or both said permeate and said concentrate to the stream of the concurrently ongoing procedure, in particular to the concurrently ongoing pulping procedure. Here, it is preferable that the LCCD composition is returned to the stream downstream of the point in the concurrently ongoing procedure, in particular of the concurrently ongoing pulping procedure, from which the initial composition was obtained.

A further aspect the invention provides a method of preparing a carbohydrate-enriched composition, comprising the steps of performing the method of processing an initial composition comprising a lignosulfonate and a carbohydrate as described herein, and collecting the resulting carbohydrate-enriched composition.

In a further embodiment of any of the methods of the invention, or embodiments thereof, described above, the method of the invention further comprises converting a carbohydrate or carbohydrates in the carbohydrate-enriched composition to a compound chemically distinct from said carbohydrate or carbohydrates. In certain embodiments, said converting is effected by a microorganism. In certain embodiments, the compound chemically distinct from said carbohydrate is chosen from the group consisting of 1,4-butanediol, lactic acid, polylactic acid, one or more one or more butenes, butadiene (1,3-butadiene), acrylic acid, polyhydroxyalkanoates, succinic acid, adipic acid, n-butanol, isobutanol, 1,2-butanediol, 2,3-butanediol, 1,3-propanediol, 1,2-propanediol, isoprene, 5-hydroxymethylfurfural (5-HMF), furfural, furanedicarboxylic acid, mannitol, sorbitol, xylitol, glucaric acid, mannaric acid, xylaric acid, gluconic acid, mannonic acid, xylonic acid, and citric acid. In an especially preferred embodiment the compound chemically distinct from said carbohydrate is 1,4-butanediol or lactic acid. The skilled person will understand that the above list of compounds chemically distinct from said carbohydrate is not exhaustive. In particular, the skilled person will understand that microorganisms may be engineered to convert carbohydrates to any number of compounds chemically distinct from carbohydrates, and that virtually any conversion of one or more carbohydrates to a compound chemically distinct therefrom may be effected given the availability of suitable genes for incorporation into said microorganisms.

A further aspect of the invention provides a carbohydrate-enriched composition produced or producible by the method of the invention, or any embodiments thereof as described herein. The carbohydrate-enriched composition produced or producible by the method of the invention, or any embodiments thereof as described herein, comprises at least one carbohydrate originally present in the initial composition.

A further aspect of the invention relates to the use of a carbohydrate-enriched composition produced or producible by the method of the invention, or any embodiments thereof as described herein, as a substrate for conversion of said carbohydrate into a compound chemically distinct from said carbohydrate. The used carbohydrate-enriched composition produced or producible by the method of the invention, or any embodiments thereof as described herein, comprises at least one carbohydrate originally present in the initial composition. In certain embodiments, said conversion is effected by a microorganism. In certain embodiments, the compound chemically distinct from said carbohydrate is chosen from the group consisting of 1,4-butanediol, lactic acid, polylactic acid, one or more butenes, butadiene (1,3-butadiene), acrylic acid, polyhydroxyalkanoates, succinic acid, adipic acid, n-butanol, isobutanol, 1,2-butanediol, 2,3-butanediol, 1,3-propanediol, 1,2-propanediol, isoprene, 5-hydroxymethylfurfural (5-HMF), furfural, furanedicarboxylic acid, mannitol, sorbitol, xylitol, glucaric acid, mannaric acid, xylaric acid, gluconic acid, mannonic acid, xylonic acid, and citric acid. In an especially preferred embodiment the compound chemically distinct from said carbohydrate is 1,4-butanediol or lactic acid. The skilled person will understand that the above list of compounds chemically distinct from said carbohydrate is not exhaustive. In particular, the skilled person will understand that microorganisms may be engineered to convert carbohydrates to any number of compounds chemically distinct from carbohydrates, and that virtually any conversion of one or more carbohydrates to a compound chemically distinct therefrom may be effected given the availability of suitable genes for incorporation into said microorganisms.

In general, the carbohydrate-enriched composition will comprise at least one carbohydrate originally present in the initial composition.

### Definitions

As used herein, the terms "carbohydrate", "sugar" and "saccharide" are used interchangeably as referring to the stated class of compounds well known to and recognized by the skilled person. These compounds are aliphatic polyhydroxycarbonyl compounds and contain at least carbon, oxygen and hydrogen, at least one carbonyl group (an aldehyde or keto group) and hydroxyl groups. The general formula of carbohydrates in their monomeric form (aldose or ketose monosaccharides) is CₙH₂ₙOₙ with n normally equal 3-8. In carbohydrate dimers (disaccharides), oligomers (oligosaccharides) and carbohydrate polymers (polysaccharides), carbohydrate monomers are linked via glycosidic (i.e. acetalic) bonds between a carbonyl and hydroxyl groups of two carbohydrate monomers. The general formula of carbohydrate polymers is (CₙHn-₂Oₙ₋₁)ₘ. In deoxy carbohydrates, one or more hydroxyl groups are replaced by a hydrogen group. In polyol carbohydrates the carbonyl group of an aldose or ketose monosaccharide is replaced by a hydroxyl group, in onic acid carbohydrates, the aldehyde group of an aldose monosaccharide is replaced by a carboxyl group, in uronic acids the terminal alcoholic hydroxyl group of a monosaccharide is replaced by a carboxyl group. The group includes monosaccharides, disaccharides, oligosaccharides and polysaccharides. For instance, a monosaccharide carbohydrate may be e.g. glucose, galactose, fructose, mannose, arabinose, rhamnose, xylose or any combination thereof; a disaccharide carbohydrate may be e.g. cellobiose, mannobiose, xylobiose, sucrose, lactose, maltose, trehalose or any combination thereof; a polyol carbohydrate may be e.g. sorbitol, mannitol or a combination thereof; an onic acid carbohydrate may be e.g. a gluconic acid, a mannonic acid, a xylonic acid or a combination thereof, an oligosaccharide carbohydrate, e.g. a carbohydrate molecule with about 3, 4, 5, 6, 7, 8, 9, 10, 11, 12, 13, 14 or 15, preferably about 3 to about 10 monosaccharide subunits, such as ß-1,4-glycoside-linked gluco-oligosaccharide a ß-1,4-glycosidic linked manno-oligosaccharide or a ß-1,4-glycosidic linked xylo-oligosaccharide, a malto-oligosaccharide such as for instance maltodextrin; a non-malto-oligosaccharide oligosaccharide carbohydrate may be e.g. raffinose, stachyose, any fructo-oligosaccharide or any combination thereof; a polysaccharide carbohydrate may for instance be a starch, for example amylose, amylopectin, a modified starch or any combination thereof, or may be a non-starch carbohydrate such as e.g. cellulose, a polyose such as a hemicellulose or a pectin.

As used herein, the term "lignosulfonate" refers to derivatives of lignin typically obtained as byproducts of wood pulping procedures employing sulfite pulping procedures. These molecules may be dimers, trimers, tetramers, pentamers, hexamers, oligomers, or polymers of units derived from phenylpropane substructures and are substituted with a sulfonic acid group on the aliphatic sidechain of some of these phenylpropane subunits and typically contain phenolic groups on the 4-position of some of the aromatic rings.

As used herein, the term "about" when referring to a measurable value such as an amount, a temporal duration, and the like, is meant to encompass, in addition to that measurable value itself, variations of ±20% or ±10%, in some instances ±9%, ±8%, ±7%, ±6%, ±5%, ±4%, ±3%, ±2% or ±1%, and in some instances ±0.9%, ±0.8%, ±0.7%, ±0.6%, ±0.5%, ±0.4%, ±0.3%, ±0.2% or ±0.1% from the specified value. It is to be understood that the term "about", in reference to a particular value, includes that exact particular value itself, irrespective of any explicit mention that that exact particular value is included. Thus, the absence of an explicit indication that the term "about" includes the particular exact recited value is not to be understood that the particular recited value is excluded from the range of variations created by the term "about"; even in the absence of an explicit indication that the term "about" includes the particular exact recited value, that exact particular value is still included in said range of variations created by the term "about". The skilled person will recognize when deviations around a stated value may be either integral or fractional (as for example for a temperature or a pressure), and when such deviations may only be integral (as for example for discrete moieties within a larger molecule). In addition to the above general definition of the term "about", this term may in particular refer to ±0.1 pH units relative to a stated pH value, ±1°C relative to a stated temperature value, ±1 bar relative to a stated pressure value, or any combination thereof.

As used herein, the term "catalyst" refers to a compound which enhances the rate of a chemical reaction by reducing the activation energy without being consumed during the chemical reaction. Such a "catalyst" is sometimes referred to as a "chemical catalyst".

As used herein, the term "biocatalyst" refers to a protein, in particular an enzyme, that acts as a catalyst in a biochemical reaction. Like a chemical catalyst, a biocatalyst enhances the rate of a chemical reaction without being consumed during the reaction. Enzymes are for example biocatalysts.

As used herein, the term "separation procedure" refers to a process which is applied to a mixture of compounds in a given composition, for instance the initial composition, and which leads to two or more fractions, where at least one compound of the original mixture is contained in either fraction.

As used herein, the term "concentration procedure" refers to a process which is applied to a compound or a mixture of compounds in a given composition, for instance the lignosulfonate-depleted composition, and which leads to two or more fractions, where at least one compound present in the original composition is found in a higher concentration - e.g. in terms of g/L - in one of the fractions than in the original composition.

As used herein, the term "concentration/removal procedure" refers to a concentration procedure as defined herein, in which said concentration simultaneously effects removal of non-carbohydrate solutes from the lignosulfonate-depleted composition which were not previously removed in the separation procedure. For example, in the event that the initial composition is part of an SSL stream of a wood or lignocellulosic pulping process, certain impurities such as furfural, 5-HMF, acetic acid, formic acid or any combination thereof generated during the pulping process may still persist in the lignosulfonate-depleted composition following the separation procedure. Subjecting this lignosulfonate-depleted composition to a subsequent concentration procedure, e.g. a concentration procedure employing a semipermeable membrane, will simultaneously concentrate the carbohydrate present in the lignosulfonate-depleted composition while also removing furfural and 5-HMF from the lignosulfonate-depleted composition, so that the carbohydrate-enriched composition resulting from the concentration/removal procedure will not only have a higher concentration of carbohydrate, but will also have a lower concentration of impurities such as furfural and 5-HMF than present in the lignosulfonate-depleted composition. The same would apply to a concentration procedure employing an evaporation step; in this event, any non-carbohydrate impurities present in the lignosulfonate-depleted composition would be removed by evaporation into the condensate along with water. The result is that the carbohydrate-enriched composition will have lower concentrations of such impurities than in the lignosulfonate-depleted composition, while at the same time it will also have a higher carbohydrate concentration than the lignosulfonate-depleted composition.

As used herein, the term "initial composition" refers to a composition comprising a lignosulfonate and a carbohydrate. The initial composition is preferably aqueous. The initial composition may be taken from or derived from a product stream of a pulping process, for example a process pulping biomass, for example a wood or lignocellulosic biomass.

As used herein, the term "**l**ignosulfonate-**c**ontaining and **c**arbohydrate-**d**epleted (LCCD) composition" refers to one fraction resulting from performing a separation procedure on an initial composition. The LCCD composition is preferably aqueous. While the product of this separation procedure still contains lignosulfonates originally present in the initial composition, although not necessarily in the same amount or concentration of lignosulfonates present in the initial composition, the amount of carbohydrate present in the LCCD composition is less than the amount of carbohydrate present in the initial composition, i.e. at least some, preferably most, more preferably all or substantially all carbohydrate has been removed from the initial composition by subjecting this initial composition to the separation procedure, to yield the LCCD composition.

As used herein, the term "lignosulfonate-depleted composition" refers to another fraction of performing a separation procedure on an initial composition. The lignosulfonate-depleted composition is preferably aqueous. The lignosulfonate-depleted composition will contain at least some, preferably most, most preferably all or substantially all of the carbohydrate originally present in the initial composition, but will contain less, preferably no or substantially no lignosulfonate originally present in the initial composition, this lignosulfonate previously having been removed in the course of the separation procedure. As such, the lignosulfonate-depleted composition contains carbohydrate.

As used herein, the term "carbohydrate-enriched composition" refers to a composition in which the concentration of carbohydrate is greater than the concentration of carbohydrate in the initial composition or in the lignosulfonate-depleted composition or in both the initial composition and the lignosulfonate-depleted composition. The carbohydrate-enriched composition is an aqueous composition. As used herein, the "carbohydrate-enriched composition" will be the result of performing the concentration procedure, optionally as a concentration/removal procedure as explained herein, on the lignosulfonate-depleted composition, so as to increase the carbohydrate concentration in the lignosulfonate-depleted composition.

As used herein, the term "pulping" or "pulping procedure" refers to a process during which biomass is treated in a way that a fibrous cellulose-containing material - for example a cellulose pulp - is formed from said biomass. The biomass may for example be wood or lignocellulosic biomass, and in this event the "pulping" or "pulping procedure" may be referred to as "wood or lignocellulosic biomass pulping" or a "wood or lignocellulosic biomass pulping procedure". Examples of specific modes of pulping include mechanical pulping, thermomechanical pulping, organosolv pulping (pulping employing an organic solvent), chemical pulping employing sulfate, soda or sulfite pulping technology known to the skilled person.

As used herein, the term "stream" refers to a process mass fraction generated continuously or semi-continuously in an industrial scale chemical, biochemical or biotechnological process. In many cases, due to the continuous or semi-continuous nature of the product generation, the "stream" often implies a directionality of flow, with locations within the flow closer to the origin of product generation being "upstream" of locations within the flow closer to the end; the latter are "downstream". For example, the term "downstream" may refer to a process taking place after a chemical, biochemical or biotechnological conversion process which has previously taken place in the ongoing product flow. Conversely, the term "upstream" may refer to a process taking place before a chemical, biochemical or biotechnological conversion process which is intended to take place later in the ongoing product flow. For instance, within certain embodiments of the present invention, the initial composition subjected to the separation procedure and concentration procedure; the separation procedure, concentration procedure and concentration/removal procedure; or the separation procedure and concentration/removal procedure of the invention, may be removed from the product stream at a point upstream of a later point in product flow at which the e.g. LCCD composition, the permeate of a concentration procedure employing a semipermeable membrane, the condensate of a concentration procedure including an evaporation step or both said permeate and said condensate is/are returned into the flow.

As used herein, the term "oligosaccharide" refers to a carbohydrate/saccharide molecule with three to ten anhydromonosaccharide units such as aldoses, ketoses, sugar acids such as glucuronic or galacturonic acids, which are linked covalently with glycosidic bonds. Alcoholic groups may be substituted with acids via ester bonds or with alcohols via ether bonds. End groups may consist of aldonic acids such as gluconic acids, xylonic acids, mannonic acids or any combination thereof.

As used herein, the term "polysaccharide" refers to a carbohydrate/saccharide molecule with eleven or more anhydromonosaccharide units such as aldoses, ketoses, sugar acids such as glucuronic or galacturonic acids, which are linked covalently with glycosidic bonds. Alcoholic groups may be substituted with acids via ester bonds or with alcohols via ether bonds. End groups may consist of aldonic acids such as gluconic acids, xylonic acids, mannonic acids or any combination thereof.

As used herein, the term "diafiltration" refers to a pressure-driven liquid/liquid separation and concentration process describing membrane separation processes in general and including ultrafiltration and nanofiltration in particular. To keep the feed volume constant, solvent is often initially continuously refilled into the portion of the composition retained behind the membrane, i.e. to the "retentate". Thus, permeating molecules passing through the membrane with solvents to form the "permeate" are further separated from the mixture but the concentration of the dissolved solute substances retained by the membrane does not increase in the retentate. Cessation of refilling of solvent into the retentate leads, upon continuation of filtration, to an increase in the concentration of solute substances in the retentive. Thus, the term "diafiltration" as used herein refers to a process which can be used to remove or lower the concentration of unwanted impurities while at the same time concentrating solute substances in the retentate. Of course, diafiltration may also refer to a process which can be used to simply concentrate solute substances in the retentate, without removing or lowering the concentration of unwanted impurities.

As used herein, the term "ultrafiltration" refers to a pressure-driven liquid/liquid separation and concentration process based on filtration employed to macromolecules dissolved in e.g. aqueous systems in the molecular mass range between about 1,000 Da and about 100,000 Da. Ultrafiltration uses a semipermeable porous membrane as a filter medium. The separation takes place according to the size of the dissolved substances. Solvent and molecules smaller than the molecular weight cut-off of the membrane permeate through the membrane and are subsequently contained in the "permeate", i.e. that portion of solvent and solute(s), which passed through the membrane. 90% of the molecules larger than the molecular weight cut-off do not permeate through the membrane but are retained, thus forming the "retentate". Due to the passage of solvent through the membrane, the concentration of solute molecules increases in the retentate.

As used herein, the term "nanofiltration" refers to a pressure driven liquid/liquid separation and concentration process based on filtration employed to molecules and ions dissolved in aqueous systems in the molecular mass range below about 1,000 Da. Nanofiltration uses diffusion membranes as a filter medium. The separation takes place according to the size of the dissolved substances. Solvent and molecules smaller than the molecular weight cut-off of the membrane permeate through the membrane and are subsequently contained in the "permeate", i.e. that portion of solvent and solute(s), which passed through the membrane. 90% of the molecules larger than the molecular weight cut-off do not permeate through the membrane but are retained, thus forming the "retentate". Due to the passage of solvent through the membrane, the concentration of solute molecules increases in the retentate.

As used herein, the term "reverse osmosis" refers to a pressure driven liquid/liquid separation and concentration process based on filtration applied to small molecules and ions dissolved in aqueous systems. Reverse osmosis uses diffusion membranes as a filter medium. Depending on the reverse osmosis membrane employed, water and some small organic molecules permeate through the membrane and are subsequently contained in the "permeate", i.e. that portion of solvent and solute(s), which passed through the membrane. Molecules which do not permeate through the membrane but are retained, form the "retentate". Due to the passage of solvent through the membrane, the concentration of solute molecules increases in the retentate.

Any aspect, embodiment or feature of the invention described hereinabove or hereinbelow may be freely combined with any other aspect, embodiment or feature of the invention described hereinabove or hereinbelow. This applies regardless of whether the possibility of such combination is explicitly stated hereinabove or hereinbelow.

The present invention will now be described in greater detail.

### Detailed description of the invention

In one aspect the invention provides a method of processing an initial composition comprising a lignosulfonate and a carbohydrate, said method comprising: subjecting the initial composition to a separation procedure whereby an amount of the lignosulfonate is removed from the initial composition, thereby yielding a **l**ignosulfonate-**c**ontaining and **c**arbohydrate-**d**epleted (LCCD) composition and a lignosulfonate-depleted composition; and subjecting the lignosulfonate-depleted composition to a concentration procedure whereby the carbohydrate concentration is increased relative to the carbohydrate concentration in the initial composition, relative to the carbohydrate concentration in the lignosulfonate-depleted composition, or relative to the carbohydrate concentration in both the initial composition and the lignosulfonate-depleted composition, thereby yielding a carbohydrate-enriched composition.

As described herein above, current technology allows the separation of lignosulfonate(s) from lignosulfonate- and carbohydrate-comprising compositions, but does not provide for carbohydrate-enriched compositions in a form suitable for use as starting materials for conversions, e.g. microorganism-based conversions of said carbohydrate(s) into further compounds differing from the substrate carbohydrate(s). As such, the present invention is a processing method entailing carbohydrate separation and concentration that overcomes all problems mentioned above and allows for operating carbohydrate separation and concentration processes in an advantageous way, e.g. tailored to the requirements of subsequent carbohydrate conversion processes. At the same time, the method of the present invention may be advantageously separated from the chemical recovery cycle of common pulping processes, e.g. as in (bi)sulfite pulp mills, e.g. Mg-(bi)sulfite pulp mills, for example in such pulp mills for pulping wood or other biomass, while still remaining integrated in the material and energy cycles of the mill. According to the method of the invention, this is achieved by producing a composition, e.g. an aqueous process stream, which is enriched in carbohydrate, and depleted (e.g. relative to the initial composition) in lignosulfonate, metal ions, e.g. Mg(2+), sulfate, sulfite, furfural, 5-HMF, acetic acid or any combination thereof. Such a carbohydrate-enriched composition can then be fed, either as a batch or as an integrated processing stream, into a carbohydrate conversion process without prior crystallization or purification of convertible carbohydrates.

The subsequent carbohydrate conversion process may comprise a fermentation step (i.e. a step in which one or more carbohydrates is/are converted into one or more substances different than said one or more carbohydrates by a microorganism, in particular a microorganism engineered to effect said conversion), a chemical conversion step (i.e. a step in which one or more carbohydrates is/are converted into one or more substances different than said one or more carbohydrates by e.g. organic synthetic reaction, for example by way of one or more chemical catalysts, e.g. metal catalysts based on Pt, Pd, Ni, Re or any combination thereof), a bioconversion step (i.e. a step in which one or more carbohydrates is/are converted into one or more substances different than said one or more carbohydrates by a biocatalyst such as an enzyme or a combination of enzymes, an enzyme and a co-factor, an enzyme and a co-factor and a co-substrate, or a combination of enzymes with one or several co-factors, with one or several co-substrates or any combination thereof), or any combination thereof. In some cases, the mircoorganism used in a fermentation may effect such conversion naturally, i.e. based on genes and corresponding enzymes it endogenously carries, i.e. the microoganism is wild type. In other cases, however, the microorganism used in a fermentation may have been altered to carry and express heterologous and/or recombinant genes allowing it to convert the substrate carbohydrate in the carbohydrate-enriched composition into one or more desired products or intermediates of value.

Advantageously, the present invention allows tailoring of the concentration and the purity of the carbohydrate in the carbohydrate-enriched composition to the needs of a subsequent carbohydrate conversion process. In particular, depending on the technology of the conversion process (e.g. batch fermentation/ batch chemical reaction, fed-batch fermentation/ fed batch chemical reaction, continuous fermentation, to name just a few examples) and the catalyst, biocatalyst or microorganism employed, different characteristics of the carbohydrate-enriched composition may be useful to render the process technically and economically feasible. For example, some microorganisms tolerate higher inhibitor concentrations than others. In this event, it may be possible to perform the concentration procedure omitting or limiting more rigorous concentration, replacing these with less rigorous concentration steps or with no further concentration steps. In general, the carbohydrate concentration required in the carbohydrate-enriched composition is a trade-off between the costs of carbohydrate concentration and product purification, so the path to a particular carbohydrate concentration in the carbohydrate-enriched composition is also flexible and may involve a number of combinations between known concentration steps involving a semipermeable membrane or evaporation, e.g. nanofiltration steps, diafiltration steps, ultrafiltration steps, reverse osmosis steps and evaporation steps. For example if the final product desired after conversion of the carbohydrate in the carbohydrate-enriched composition is highly volatile (e.g. ethanol) or insoluble in water (e.g. butanol), then lower carbohydrate concentrations in the carbohydrate-enriched composition may be sufficient. In contrast, if the final product desired after conversion of the carbohydrate in the carbohydrate-enriched composition is a product of higher boiling point such as 1,4-butanediol, it may be necessary to perform a more extensive concentration procedure according to the present invention to achieve higher carbohydrate concentrations in the carbohydrate-enriched composition, thus rendering purification, e.g. by distillation, of the final product following carbohydrate conversion more economically feasible. What is feasible or not depends also on the energy costs on site, and this is known to the skilled person. The skilled person will thus be able to combine separation and concentration steps as desired in order to result in a final concentration of carbohydrate in the carbohydrate-enriched composition which fits with the ultimate downstream objective.

The inventive processing method is a process comprising a sequence of at least two operations in a novel and defined sequence. The sequential performance of these steps serves the purpose described above and entails several advantages. In particular, in a first step the initial composition comprising a lignosulfonate and a carbohydrate is subjected to a separation procedure whereby an amount of the lignosulfonate is removed from the initial composition. The result, obtained in at least two fractions, is a **l**ignosulfonate-**c**ontaining and **c**arbohydrate-**d**epleted (LCCD) composition and a lignosulfonate-depleted composition. As will be explained in more detail below, the LCCD composition may be fed back into the source from which the initial composition was drawn, for example back into the continuous stream, e.g. SSL-stream of a pulping process, e.g. a wood or other biomass pulping process, so that the lignosulfonate removed from the initial composition by the separation procedure may be recovered downstream, either as lignosulfonate *per se* (as lignosulfonates constitute valuable products in their own right), or as a source of valuable sulfur, which can be subsequently recovered as SO₂ from the combustion gases following burning, as described above. The amenability of the inventive method to integration as an independent loop within an existing process stream of an initial composition constitutes one advantage of the invention.

A further advantage of the invention is however to be seen in the stepwise removal of substances from the initial composition which are potentially disturbing for desired downstream carbohydrate conversion(s). The separation of the inventive method into two procedural phases - an initial one of separation and a subsequent one of concentration (and possible concomitant removal of further substances by way of such concentration) - allows the sequential removal of substances from the initial composition under different conditions suited for the various disturbing substances. Rather than being bound to one set of treatment conditions which may remove some disturbing substances while leaving others behind, the inventive method therefore allows directed tuning of the conditions (as specified in greater detail herein) employed in each of the process steps to optimally remove each of the disturbing substances in different procedural phases. The result is a final carbohydrate-enriched composition, preferably a final aqueous carbohydrate-enriched composition, which contains very little or no substances which could potentially disturb or impede intended downstream conversion of carbohydrate, and a maximum amount of carbohydrate for such conversion itself. It is therefore not the purpose of the invention to achieve a purified carbohydrate stream from which pure carbohydrates can be recovered e.g. in crystalline form. In fact, it is particularly preferred that the carbohydrate in the carbohydrate-enriched composition, not be crystallized, in particular not be crystallized subsequent to the concentration procedure, and that the carbohydrate-enriched composition is aqueous. Crystallization of carbohydrate generally requires prior removal of carbohydrate crystallization inhibitors from the lignosulfonate-depleted composition, from the carbohydrate-enriched composition or from both the lignosulfonate-depleted composition and the carbohydrate-enriched composition, and the removal of such inhibitors (themselves often carbohydrates) reduces net carbohydrate yield. The avoidance of crystallization in the inventive method has the advantage that one avoids such losses in carbohydrate yield. Rather, the inventive method achieves a tailored stream to meet the requirements of a carbohydrate conversion process in terms of e.g. yield, rate, volumetric rate and conversion product concentration and total energy demand.

In the first step of the method of the present invention (the separation procedure), cooking chemicals such as metal salt complexes of lignosulfonates (e.g. Mg-lignosulfonates) and optionally metal salt complexes of sulfites and sulfates (e.g. Mg-sulfites and Mg-sulfates) are separated from carbohydrates, while at the same time increasing the water:carbohydrate ratio in the carbohydrate stream at no or minimal dilution of the feed carbohydrate concentration [g/L]. The increase in the water:carbohydrate ratio without substantially reducing the carbohydrate concentration in the initial composition results from the replacement of lignosulfonates and other solids present in the initial composition with water. This removal of lignosulfonates has the effect of reducing the load of toxic phenolic compounds (lignosulfonates) and sulfur compounds in the processed carbohydrate stream, i.e. in the lignosulfonate-depleted composition. Lignosulfonate removal also has the effect that the viscosity of the lignosulfonate-depleted composition is lower than that of the initial composition, thus making more feasible a subsequent concentration step aimed at increasing the carbohydrate concentration above that in the initial composition, in the lignosulfonate-depleted composition or in both the initial composition and the lignosulfonate-depleted composition.

The second step, i.e. the concentration procedure which is then performed on the lignosulfonate-depleted composition resulting from the separation procedure can then be performed with greater flexibility, entailing a greater ability to tailor the carbohydrate concentration in the final carbohydrate-enriched composition to the needs of any subsequent carbohydrate conversion process. In the types of concentration steps described herein, e.g. separation employing a semipermeable membrane, evaporation or both a semipermeable membrane and evaporation, the removal of water and other volatile substances (e.g. by evaporation) or permeating substances (e.g. by use of a semipermeable membrane) can contribute to further concentration of the stream and at the same time can contribute to removal of any inhibitor compounds present in the initial composition. This is because concentration processes employing semipermeable membranes can be performed to achieve higher concentration factors and concentration processes employing evaporation can be performed to achieve higher degrees of volume reduction. Lignosulfonates are both non-volatile and non-permeating, so the lignosulfonates present in the initial composition must first be removed in the separation procedure to generate a lignosulfonate-depleted composition, before concentrating this lignosulfonate-depleted composition in a subsequent concentration procedure, to result in a carbohydrate-enriched composition.

Through removal of lignosulfonates present in the initial composition in the separation procedure, the water fraction in the lignosulfonate-depleted composition is increased, while the viscosity of the lignosulfonate-depleted composition is decreased. This is further advantageous for the subsequent concentration procedure performed on the resulting lignosulfonate-depleted composition, resulting in greater downstream processing flexibility in subsequent concentration. First, the higher water content in the lignosulfonate-depleted composition provides a greater initial amount of solvent to be removed, this afforing the opportunity to remove a greater fraction of undesired solute substances. Second, the lower viscosity resulting from the increased water fraction in the lignosulfonate-depleted composition has the effect that the lignosulfonate-depleted composition can be filtered more easily over a semipermeable membrane. In the event that a semipermeable membrane is used as part of the subsequent concentration procedure, this increased flow across the membrane also advantageously influences the extent to which unwanted impurities can be removed from the lignosulfonate-depleted composition to yield the carbohydrate-enriched composition.

In one advantageous embodiment of the method of the invention, the carbohydrate in the carbohydrate-enriched composition is not crystallized, e.g. subsequent to the concentration procedure. As mentioned above, the methods of the present invention are not intended to produce carbohydrates in pure form for further consumption of pure carbohydrates as end products. Rather, the advantage of the methods of the present invention is that the resulting carbohydrate-enriched composition, which in most cases will be a composition of mixed carbohydrates, remains solubilized, that is in aqueous form, thus retaining a form suitable for further use in downstream processes for converting the (mixed) carbohydrate into further products of interest and value. It is to be noted that even minimal or trace amounts of impurities in the carbohydrate-enriched composition may be acceptable or in some cases even useful or desirable, and that carbohydrates in the composition may be a mixture of various carbohydrates as long as the carbohydrate remains in solubilized form in the carbohydrate-enriched composition, since only then will it generally remain accessible to downstream conversion processes, such as for example downstream conversion processes which depend, in whole or in part, on the action of microorganisms, biochemical reactions, chemical reactions or any combination thereof capable of converting a carbohydrate or carbohydrates in the carbohydrate-enriched composition into further products of interest or value.

In certain embodiments, the content of glucose, arabinose or the combined content of glucose and arabinose in the lignosulfonate-depleted composition, the carbohydrate-enriched composition or both the lignosulfonate-depleted composition and the carbohydrate-enriched composition may be greater than 10 wt% based on the weight of dry solids of the respective compositions.

In certain embodiments, the content of xylose in the lignosulfonate-depleted composition, the carbohydrate-enriched composition or both may be below 50 wt%, or greater than 70 wt%, or between 50 wt% and 70 wt% based on the weight of dry solids of the respective composition or compositions, with the optional proviso that the content of xylose in the lignosulfonate-depleted composition, the carbohydrate-enriched composition or both is not 53.7 wt%, 58 wt%, 59 wt%, 59.2 wt%, 60 wt%, 61.1 wt%, 64.7 wt%, 65 wt%, 65.5 wt%, 66.1 wt%, 66.6 wt% or 67 wt%, or any combination thereof, based on the weight of dry solids of the respective composition or compositions.

In certain embodiments the xylose content of the initial composition may be below 14 wt% based on the weight of dry solids. In certain embodiments the xylose content of the initial composition may be greater than 27 wt%, with the optional proviso that the xylose content of the initial composition is not 27.3 wt%. In certain embodiments the xylose content of the initial composition may be between 14 wt% and 27 wt% based on the weight of dry solids in the initial composition, with the optional proviso that the xylose content of the initial composition is not 15.8 wt%, 17 wt%, 17.2 wt%, 24 wt% or 24.9 wt%, or any combination thereof, based on the weight of dry solids in the initial composition.

In certain embodiments the initial composition may have a dry weight content of less than 20 g per 100 g of initial composition, greater than 55 g per 100 g of initial composition, or between 20 g and 55 g per 100 g of initial composition, with the optional proviso that the initial composition does not have a dry weight content of 25 g per 100 g of initial composition, 35 g per 100 g of initial composition, or 45 g per 100 g of initial composition, or any combination thereof. In certain embodiments, the dry weight content of the initial composition refers to the dry weight content of total carbohydrate in the initial composition relative to the total dry weight in the initial composition. In certain embodiments the initial composition may have a dry weight content of total carbohydrate, based on total dry weight content of the initial composition, which is not 21.1 g per 100 g of initial composition (21.1 wt%), 23.8 g per 100 g of initial composition (23.8 wt%), 29.8 g per 100 g of initial composition (29.8 wt%), 31.9 g per 100 g of initial composition (31.9 wt%), 32 g per 100 g of initial composition (32 wt%) or 35 g per 100 g of initial composition (35 wt%) or any combination thereof.

In certain embodiments, the carbohydrate-enriched composition may have a dry weight of total carbohydrate, i.e. a dry solids content of total carbohydrate of greater than 60 wt%, with the optional proviso that the carbohydrate-enriched composition does not have a dry weight of total carbohydrate, i.e. a dry solids content of total carbohydrate of 65 wt%, 68.7 wt%, 70 wt%, 72.1 wt%, 74.1 wt%, 74.7 wt%, 75.1 wt%, 75.3 wt% or 75.9 wt% or any combination thereof.

In one embodiment, the initial composition comprising a lignosulfonate and a carbohydrate, e.g. an SSL feed obtained from a pulping process, e.g. from a (bi)sulfite pulping process, may be filtered prior to the separation procedure in order to remove non-dissolved, e.g. insoluble or colloidal particles from the composition before subjecting the initial composition to the separation procedure. This has the advantage of increasing the runability of the subsequent separation and concentration procedures, since potentially disturbing solids will have been previously removed by such filtration.

In one particularly advantageous embodiment of the method of the invention, the separation procedure is a chromatographic separation procedure. This first step can for example be accomplished by means of ion exchange chromatography, for example with a strong acid cation exchange resin in the form corresponding to the metal ion used in the cooking chemicals in the pulping process leading to the initial composition, e.g. the lignosulfonate- and carbohydrate-containing SSL. In many cases, the metal ion employed will be Mg2+ or Ca2+. In many instances, Mg2+ will be preferred, so in such cases the strong acid cation exchange resin will be in the form of Mg2+. Suitable resins may be resins known to the skilled person, for example divinylbenzene (DVB) crosslinked polystyrenesulfonate resins such as DOWEX® 99 or Finex® CS GC, or Amberlyst type chromatography resins. This chromatography in the separation procedure may be run as a multi-column recycling chromatography, for example a simulated moving bed chromatography or dual column chromatography with water as the mobile phase. The chromatographic aspect of the separation procedure, including all variants thereof may be combined with any aspect or embodiment of the present invention described herein.

The operation pH for operation of the chromatographic separation procedure may advantageously be in the typical pH range of the SSL. For instance, it is advantageous to perform the separation procedure, in particular the chromatographic separation procedure, at a pH in the range of about 2 to about 5, or at a pH in the range between about 2 and about 5, preferably at a pH in the range of about 3 to about 4, or at a pH in the range between about 3 and about 4. For instance, the separation procedure, in particular the chromatographic separation procedure may be performed at a pH of about 2.0 (including 2.0), about 2.1, about 2.2, about 2.3, about 2.4 (including 2.4), about 2.5 (including 2.5), about 2.6, about 2.7, about 2.8, about 2.9, about 3.0 (including 3.0), about 3.1, about 3.2, about 3.3 (including 3.3), about 3.4, about 3.5 (including 3.5), about 3.6, about 3.7, about 3.8, about 3.9, about 4.0, about 4.1, about 4.2, about 4.3, about 4.4, about 4.5, about 4.6, about 4.7, about 4.8, about 4.9 or about 5.0, or any pH range defined, i.e. bracketed by or between any two of these pH values. Of course, any pH range that is defined by the above mentioned pH values, including the above mentioned pH range endpoints, is also contemplated, either including or excluding the respective endpoints. Also contemplated in all of the above pH ranges are corresponding pH ranges in which the lower respective endpoint is included while the upper respective endpoint is excluded, as well as pH ranges in which the lower respective endpoint is excluded while the upper respective endpoint is included. In certain embodiments, the chromatographic separation procedure may also optionally be performed while avoiding pH values of 1, 2, 2.4, 2.5, 3.0, 3.3, 3.5 or any combination thereof.

The separation procedure, in particular the chromatographic separation procedure, is typically performed at a temperature of about 25°C to about 90°C, or at a temperature between about 25°C and about 90°C, preferably at a temperature of about 60°C to about 75°C or at a temperature between about 60°C and about 75°C. For instance, the separation procedure, in particular the chromatographic separation procedure may be performed at a temperature of about 25°C, about 30°C, about 35°C, about 40°C, about 45°C, about 50°C, about 55°C (including 55°C), about 60°C (including 60°C), about 61°C, about 62°C, about 63°C, about 64°C, about 65°C (including 65°C), about 66°C, about 67°C, about 68°C, about 69°C, about 70°C (including 70°C), about 71°C, about 72°C, about 73°C, about 74°C, about 75°C, about 80°C, about 85°C or about 90°C or any temperature range defined, i.e. bracketed by or between any two of these temperature values. Of course, any temperature range that is defined by the above mentioned temperature values, including the above mentioned temperature range endpoints, is also contemplated, either including or excluding the respective endpoints. Also contemplated in all of the above temperature ranges are corresponding temperature ranges in which the lower respective endpoint is included while the upper respective endpoint is excluded, as well as temperature ranges in which the lower respective endpoint is excluded while the upper respective endpoint is included. In certain embodiments, the chromatographic separation procedure may also be optionally performed while avoiding temperature values of 55°C, 60°C, 65°C, 70°C or any combination thereof.

As a binary separation process, chromatography of the initial composition comprising a lignosulfonate and a carbohydrate will typically lead to two main fractions. One fraction, *Fraction 1*, is an LCCD composition containing the main share of lignosulfonates, metal ions, e.g. Mg ions, and anions of mineral acids such as sulfate and sulfite previously in the initial composition. As mentioned above, this Fraction 1 (LCCD composition) can advantageously be fed back to the recovery cycle processing stream, e.g. of the (bi)sulfite pulp mill, e.g. Mg(bi)sulfite pulp mill. This return into the recovery cycle may take the form of returning the LCCD composition back into the source of the initial composition (e.g. the SSL stream), or may take the form of feeding the LCCD composition into a subsequent evaporation plant. It is also envisioned that the LCCD composition may be recycled through the separation procedure, or a further separation procedure, e.g. the or another chromatographic separation procedure, thus becoming, in such a recycling, the initial composition in a second round of separation according to the claimed method. Such recycling has the advantage that any lignosulfonate not removed from the first round of the separation procedure may be removed from the second round of the separation procedure. In this event, respective lignosulfonate-depleted compositions from first and second rounds of the separation procedure may be pooled prior to the subsequent concentration procedure. In a similar way, the respective LCCD compositions from first and second rounds of the separation procedure may be pooled before proceeding further.

Another fraction, *Fraction 2,* resulting from the separation when the initial composition is taken from e.g. an SSL stream is a lignosulfonate-depleted composition containing, besides the main share of the mixed carbohydrate compounds (e.g. xylose, mannose, glucose, galactose, arabinose, rhamnose, disaccharides consisting thereof, oligosaccharides consisting thereof), organic acids, furfural, and 5-HMF. According to the method of the invention, this aqueous lignosulfonate-depleted composition is then further processed in a subsequent concentration procedure, which may take the form of a separate operation unit within the integrated processing method, and which is described in more detail below.

In one embodiment, the concentration procedure may employ a semipermeable membrane; an evaporation step; or a semipermeable membrane and an evaporation step. In the event that the concentration procedure comprises multiple steps, there is no particular restriction on the number of concentration steps, or on the order in which they may be performed. For example, it may be advantageous to perform multiple concentration filtrations through a semipermeable membrane, continually adding solvent to the retentate in order to remove, e.g. extract the greatest amount of unwanted substances such as e.g. organic acids, furfural and 5-HMF. This repeated dilution and subsequent extraction may take the form of e.g. a diafiltration, e.g. an ultrafiltration or nanofiltration. This embodiment also includes the possibility of performing multiple consecutive concentrations over multiple semipermeable membranes of the same or different molecular weight cutoffs, with the retentate of a previous concentration step forming the substrate for a respective subsequent concentration step. Additionally, or alternatively, the concentration procedure may employ an evaporation step in which the water and the major amount of volatile contaminants, e.g. organic acids, furfural, or any combination thereof are evaporated under applied heat, reduced pressure, or a combination thereof, thereby simultaneously increasing the concentration of carbohydrate in the lignosulfonate-depleted composition and removing substances which may potentially contaminate downstream processing steps, e.g. downstream steps for microbial conversion of carbohydrate(s) in the carbohydrate-enriched composition.

In the event the concentration procedure entails the use of a semipermeable membrane, those skilled in the art will select a membrane that allows retention of the main share of carbohydrates and certain organic acids (sugar acids such as xylonic, gluconic or mannonic acid) in the retentate, while allowing a certain share of other organic acids (such as acetic acid), and other compounds such as furfural, and 5-HMF to permeate the membrane in the permeate. As mentioned above, to further reduce the concentration of permeating compounds in the retentate e.g. potential inhibitors of downstream fermentation/carbohydrate conversion such as organic acids, furfural, 5-HMF or any combination thereof, the membrane separation employing the semipermeable membrane can be processed as a diafiltration with water or other desired solution, e.g. an aqueous solution such as an aqueous carbohydrate solution, e.g. if carbohydrates from other (industrial) sources should be integrated into a downstream conversion process. By means of this membrane separation process, the carbohydrate concentration in the retentate stream (i.e. the processed lignosulfonate-depleted composition) can be increased and adjusted in the desired range. Further, by means of diafiltration performed on the lignosulfonate-depleted composition in a concentration procedure employing a semipermeable membrane, the concentration of inhibitors can be adjusted to be in a desired range i.e. below a desired threshold. Suitable membranes include for example polyamide thin film composite membranes (e.g. DOW Filmtec NF90; Oltremare NANO9, DOW Filmtec NF270, Alpha Laval RO90, RO98, and RO99; Koch MPF-34, AMS A-3010, AMS A-4022 to name but a few).

In the event that the concentration procedure employs a semipermeable membrane, this concentration procedure employing this membrane may typically be carried out in a pH range at or below about 4.5. For instance, it is advantageous to perform the membrane filtration at a pH in the range of about 2 to about 4, or at a pH in the range between about 2 and about 4, preferably at a pH in the range of about 3 to about 4, or at a pH in the range between about 3 and about 4. For instance, the membrane filtration may be performed at a pH of about 2.0, about 2.1, about 2.2, about 2.3, about 2.4, about 2.5, about 2.6, about 2.7, about 2.8, about 2.9, about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5 or any pH range defined, i.e. bracketed by or between any two of these pH values. Of course, any pH range that is defined by the above mentioned pH values, including the above mentioned pH range endpoints, is also contemplated, either including or excluding the respective endpoints. Also contemplated in all of the above pH ranges are corresponding pH ranges in which the lower respective endpoint is included while the upper respective endpoint is excluded, as well as pH ranges in which the lower respective endpoint is excluded while the upper respective endpoint is included.

The concentration procedure employing a semipermeable membrane may typically be performed at a temperature of about 15°C to about 90°C, or at a temperature between about 15°C and about 90°C, preferably at a temperature of about 35°C to about 70°C or at a temperature between about 35°C and about 70°C. For instance, the concentration procedure employing a semipermeable membrane may be performed at a temperature of about 15°C, about 20°C, about 25°C, about 35°C, about 36°C, about 37°C, about 38°C, about 39°C, about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, about 60°C, about 61°C,about 62°C, about 63°C, about 64°C, about 65°C, about 66°C, about 67°C, about 68°C, about 69°C, about 70°C, about 75°C, about 80°C, about 85°C or about 90°C or any temperature range defined, i.e. bracketed by or between any two of these temperature values. Of course, any temperature range that is defined by the above mentioned temperature values, including the above mentioned temperature range endpoints, is also contemplated, either including or excluding the respective endpoints. Also contemplated in all of the above temperature ranges are corresponding temperature ranges in which the lower respective endpoint is included while the upper respective endpoint is excluded, as well as temperature ranges in which the lower respective endpoint is excluded while the upper respective endpoint is included.

The concentration procedure employing a semipermeable membrane may typically be performed at a pressure of about 5 bar to about 60 bar, or at a pressure between about 5 bar and about 60 bar, preferably at a pressure of about 20 bar to about 50 bar or at a pressure between about 20 bar and about 50 bar. For instance, the concentration procedure employing a semipermeable membrane may be performed at a pressure of about 5 bar, about 10 bar, about 15 bar, about 20 bar, about 21 bar, about 22 bar, about 23 bar, about 24 bar, about 25 bar, about 26 bar, about 27 bar, about 28 bar, about 29 bar, about 30 bar, about 31 bar, about 32 bar, about 33 bar, about 34 bar, about 35 bar, about 36 bar, about 37 bar, about 38 bar, about 39 bar, about 40 bar, about 41 bar, about 42 bar, about 43 bar, about 44 bar, about 45 bar, about 46 bar, about 47 bar, about 48 bar, about 49 bar, about 50 bar, about 55 bar or about 60 bar or any pressure range defined, i.e. bracketed by or between any two of these pressure values. Of course, any pressure range that is defined by the above mentioned pressure values, including the above mentioned pressure range endpoints, is also contemplated, either including or excluding the respective endpoints. Also contemplated in all of the above pressure ranges are corresponding pressure ranges in which the lower respective endpoint is included while the upper respective endpoint is excluded, as well as pressure ranges in which the lower respective endpoint is excluded while the upper respective endpoint is included.

The concentration procedure employing an evaporation step may typically be performed at a temperature of about 15°C to about 90°C, or at a temperature between about 15°C and about 90°C, preferably at a temperature of about 35°C to about 70°C or at a temperature between about 35°C and about 70°C. For instance, the concentration procedure employing an evaporation step may be performed at a temperature of about 15°C, about 20°C, about 25°C, about 35°C, about 36°C, about 37°C, about 38°C, about 39°C, about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, about 60°C (including 60°C), about 61°C, about 62°C, about 63°C, about 64°C, about 65°C (including 65°C), about 66°C, about 67°C, about 68°C, about 69°C, about 70°C, about 75°C, about 80°C, about 85°C or about 90°C or any temperature range defined, i.e. bracketed by or between any two of these temperature values. In certain embodiments, the concentration procedure employing an evaporation step may also be performed while avoiding a temperature of 60°C or 65°C or any combination thereof. Of course, any temperature range that is defined by the above mentioned temperature values, including the above mentioned temperature range endpoints, is also contemplated, either including or excluding the respective endpoints. Also contemplated in all of the above temperature ranges are corresponding temperature ranges in which the lower respective endpoint is included while the upper respective endpoint is excluded, as well as temperature ranges in which the lower respective endpoint is excluded while the upper respective endpoint is included.

The skilled person understands that flow is a result of transmembrane pressure, temperature and state of the membrane (e.g. type of membrane, composition of the flow, prior operation time, extent of any membrane fouling). Other diluents besides water might include aqueous carbohydrate solutions from other sources than the initial composition, e.g. SSL, as well as suitable aqueous streams derived from a pulp mill. Biorefinery operations could be integrated accordingly. Combinations of these aqueous streams could be used.

As mentioned above, the permeate may advantageously be fed to the pulp mill e.g. for pulp washing or into an anaerobic waste water treatment or in a device for acetic acid and furfural separation and purification such as vapor condensate extraction. The retentate is a carbohydrate-enriched composition in which the carbohydrate concentration is increased relative to the carbohydrate concentration in the initial composition, relative to the carbohydrate concentration in the lignosulfonate-depleted composition or relative to the carbohydrate concentration in the initial composition and in the lignosulfonate-depleted composition. This purified and concentrated aqueous carbohydrate stream, i.e. carbohydrate-enriched composition, can be fed directly into a carbohydrate conversion process, can be subjected to a further concentration procedure employing a semipermeable membrane, e.g. a semipermeable membrane with a different molecular weight cutoff than the semipermeable membrane in the preceding concentration step, or can be further concentrated in an evaporation unit as a further step in the concentration procedure. Of course, the order of the steps within the concentration procedure is not fixed, and may be varied as appropriate to obtain a carbohydrate-enriched composition having a carbohydrate concentration greater than that in the lignosulfonate-depleted composition, in the initial composition or in the lignosulfonate-depleted composition and in the initial composition, while reducing unwanted substances such as e.g. organic acids, furfural, 5-HMF or any combination thereof to a minimum.

In certain advantageous embodiments, the concentration procedure comprises at least a semipermeable membrane, which may be employed either alone or as part of a broader concentration procedure also including another semipermeable membrane, an evaporation step, or both.

In the event that the concentration procedure employs an evaporation step, this step may be performed alone, as the only step in the concentration procedure, or alternatively may be performed together with one or more steps of the concentration procedure employing a semipermeable membrane, a further evaporation step or steps or together with one or more steps of the concentration procedure employing a semipermeable membrane and a further evaporation step or steps. In the event that an evaporation step follows a concentration step involving a semipermeable membrane, the retentate of the membrane separation unit is advantageously fed into a downstream evaporation unit. That is, in the event that the concentration procedure includes an evaporation step, the lignosulfonate-depleted composition resulting from the separation procedure (or, alternatively, the retentate obtained from a preceding concentration procedure employing a semipermeable membrane) is advantageously fed to an evaporation unit for (further) concentration. Those skilled in the art will select a layout of units allowing membrane-based and/ or evaporative concentration to achieve carbohydrate concentration and separation of non-carbohydrate compounds from the mixed carbohydrate stream.

In the event the concentration procedure includes an evaporation step, the carbohydrate mixture, certain organic acids and residual salts are thickened, while residual volatile acids (e.g. acetic acid) and furfural are evaporated to some extent together with water and are thus found in the condensate resulting from the evaporation, e.g. in the evaporation unit. The condensate can be fed back into the processing stream, e.g. of the pulp mill, e.g. for pulp washing or into an anaerobic waste water treatment or in a device for acetic acid and furfural separation and purification such as vapor condensate extraction. The evaporate, which is a purified and concentrated aqueous carbohydrate stream can be fed into a carbohydrate conversion process, or can be subjected to further concentration steps, e.g. comprising a semipermeable membrane, a further evaporation or a semipermeable membrane and a further evaporation, before finally being fed into a downstream carbohydrate conversion process.

In the evaporation unit, the carbohydrate mixture, certain organic acids and residual salts are thickened, while volatile acids, and furfural are evaporated to some extent together with water and are thus found in the condensates of the evaporation unit. The condensate can be fed to the pulp mill e.g. for pulp washing or into an anaerobic waste water treatment or in a device for acetic acid and furfural separation and purification such as vapor condensate extraction. The evaporate (i.e. the carbohydrate-enriched composition) represents a purified and concentrated aqueous carbohydrate stream which can be fed into a downstream carbohydrate conversion process as previously discussed. As also previously discussed, the evaporate (i.e. the carbohydrate-enriched composition) may also be subsequently subjected to further concentration steps as described hereinabove, e.g. concentration procedure(s) employing a semipermeable membrane or further evaporation steps.

If performed, the evaporation procedure may be performed according to parameters known to the skilled person. Generally, temperatures should be chosen so as to allow evaporation of unwanted substances from the lignosulfonate-depleted composition, while at the same time avoiding temperatures which are so high that desired carbohydrates in the lignosulfonate-depleted composition might be degraded. For this purpose, one will typically perform concentration by evaporation at temperatures below about 120°C. To avoid such high temperatures while at the same time ensuring removal of solvent, removal of unwanted impurities or removal of solvent and of unwanted impurities from the lignosulfonate-depleted composition, it may for example be advantageous to perform the concentration procedure by evaporation at reduced pressure with no, or at least less, applied heat. The particular combination of temperature and pressure to be employed in the evaporation concentration will typically depend on multiple factors such as e.g. the content of the lignosulfonate-depleted composition, the energy exchange, and the equipment used.

Typically, the concentration procedure is adjusted or performed long enough, or performed with sufficient repetition so as to result in a carbohydrate concentration in the carbohydrate-enriched composition of at least about 200 g/L, at least about 300 g/L, at least about 400 g/L at least about 500 g/L, at least about 600 g/L or at least about 700 g/L, or at least about 800 g/L. Advantageously, the concentration procedure will be adjusted, repeated or adjusted and repeated so as to ensure a carbohydrate concentration in the carbohydrate-enriched composition of at least about 510 g/L, at least about 520 g/L, at least about 530 g/L, at least about 540 g/L, at least about 550 g/L, at least about 560 g/L, at least about 570 g/L, at least about 580 g/L, at least about 590 g/L, at least about 600 g/L (including at least 600 g/L), at least about 610 g/L, at least about 620 g/L, at least about 630 g/L, at least about 640 g/L, at least about 650 g/L (including at least 650 g/L), at least about 660 g/L, at least about 670 g/L, at least about 680 g/L, at least about 690 g/L, at least about 700 g/L (including at least 700 g/L), at least about 710 g/L, at least about 720 g/L, at least about 730 g/L, at least about 740 g/L, at least about 750 g/L, at least about 760 g/L, at least about 770 g/L, at least about 780 g/L, at least about 790 g/L or at least about 800 g/L. It is to be understood that the lignosulfonate-depleted composition, like the final carbohydrate-enriched composition, will normally be an aqueous composition, as is clear from the units of "g/L" indicated above. Of course, any concentration range that is defined by the above mentioned concentration values, including the above mentioned concentration range endpoints, is also contemplated, either including or excluding the respective endpoints. Also contemplated in all of the above concentration ranges are corresponding concentration ranges in which the lower respective endpoint is included while the upper respective endpoint is excluded, as well as concentration ranges in which the lower respective endpoint is excluded while the upper respective endpoint is included. In certain optional embodiments, it is possible that the dry solids content of carbohydrate in the carbohydrate-enriched composition is not 600 g/kg (60 wt%), 650 g/kg (65 wt%), 700 g/kg (70 wt%), 820 g/kg (82 wt%), 830 g/kg (83 wt%) or 860 g/kg (86 wt%).

In one embodiment, the carbohydrate comprises xylose, mannose, glucose, galactose, arabinose, rhamnose, ribose, fucose or any combination thereof; disaccharides, oligosaccharides or polysaccharides comprising any thereof or combination thereof; onic acids; uronic acids; hemicelluloses; hydrolysis products of hemicelluloses; or any combination thereof. Such carbohydrates will typically be present as part of wood or lignocellulosic material pulping processes. Accordingly, in one embodiment of the method of the invention, the initial composition is a composition resulting from pulping or pretreating a lignocellulosic material, such as wood, preferably using H₂SO₃, solutions of SO₂ or both. In such cases, the initial composition will typically be but need not necessarily be a spent sulfite liquor (SSL).

In one embodiment of the method of the invention, the initial composition comprising the lignosulfonate and the carbohydrate is obtained from the stream of a concurrently ongoing procedure. As explained above, the concurrently ongoing procedure may be part of a wood or lignocellulosic biomass pulping process, e.g. a (bi)sulfite wood or lignocellulosic biomass pulping process, e.g. a Mg-(bi)sulfite wood or lignocellulosic biomass pulping process, and the initial composition may be a SSL resulting from pulping or pretreating a lignocellulosic material such as wood, for example using H₂SO₃, solutions of SO₂ or both. In this case, the method of the invention can be advantageously implemented by additionally returning the LCCD composition resulting from the separation procedure to the stream of the concurrently ongoing procedure, e.g. to the SSL stream from which the initial composition was drawn. The same applies to the permeate of the concentration procedure employing a semipermeable membrane, to the condensate of the concentration procedure employing an evaporation step or to said permeate and said condensate; either or each of these solutions may be returned to the stream of the concurrently ongoing procedure, e.g. to the SSL stream from which the initial composition was drawn. Ideally, the LCCD composition, the permeate of the concentration procedure employing a semipermeable membrane, the condensate of the concentration procedure employing an evaporation step or said permeate and said condensate are advantageously returned to one of the streams of the concurrently ongoing procedure, e.g. to the SSL stream, at a point downstream of the point in the concurrently ongoing procedure from which the initial composition was obtained/drawn or is being obtained/drawn. This recycling of the substances removed from the initial composition throughout the steps of the method of the invention advantageously allows access to compounds of potential value in their own right, or additional energy from compounds to be harnessed in subsequent burning, at the end of the pulping cycle e.g. the wood or lignocellulosic biomass pulping cycle. That is, the present invention allows flexible integration into an established pulping process e.g. a wood or lignocellulosic biomass pulping process as an independent, parallel loop diverted from the processing stream, which opens access to aqueous carbohydrate-enriched compositions ideally suited for feeding into subsequent carbohydrate conversion procedures, while preventing undesirable loss of potentially valuable substances removed from the initial composition. In this way, the method of the invention advantageously provides a way of achieving two objectives simultaneously - accessing a carbohydrate-enriched composition to allow downstream conversion of carbohydrates into further products of value, while avoiding unneeded waste of other substances which, though they may be disturbing to such intended downstream carbohydrate processing, still represent products of value in their own right. Further, all of this is achieved while avoiding the need for significant modifications in existing processes, e.g. in an existing wood or biomass pulping plant, as the steps of the inventive method lend themselves well to modular integration orthogonal to an existing processing stream.

In a further aspect the invention provides a method of preparing a carbohydrate-enriched composition comprising the steps of performing the processing method as described hereinabove, and collecting the resulting carbohydrate-enriched composition. As explained above, a major advantage of the subsequent chaining of a separation procedure and a concentration procedure according to the method of the invention allows the production of a carbohydrate-enriched composition ideally suited as a substrate for subsequent procedures aimed at the conversion of the carbohydrate or carbohydrates in the carbohydrate-enriched composition into other products of value which are chemically distinct from the carbohydrate or carbohydrates in the carbohydrate-enriched composition. The carbohydrate-enriched composition is ideally suited for use as such a substrate, because the combination of the separation and concentration procedures as described hereinabove has the net effect of removing substances which could potentially inhibit such a downstream conversion, while increasing the carbohydrate concentration so that such downstream conversions of the carbohydrate into other products are economically feasible. As mentioned above, avoidance of a carbohydrate crystallization step in the inventive method also has the advantageous effect of avoiding a decrease in carbohydrate yield which would otherwise ensue due to removal of carbohydrates which would otherwise act as crystallization inhibitors for any desired carbohydrate to be crystallized. The avoidance of a carbohydrate crystallization step in the inventive method also ensures that the resulting carbohydrate-enriched composition will remain in a soluble, e.g. aqueous form which is amenable for use in a subsequent carbohydrate conversion process.

The present aspect of the invention, providing a method of preparing the carbohydrate-enriched composition, and comprising the step of collecting the resulting carbohydrate-enriched composition resulting from the above processing steps, is highly advantageous because it allows the resulting carbohydrate-enriched composition to be transiently stored, collected or stored and collected prior to entry into subsequent conversion processes. In some instances, it may be desirable to collect a certain amount of carbohydrate-enriched composition before initiating desired carbohydrate conversion into other products, i.e. it may be desirable to run the conversion process in a batch-wise mode rather than continuously. In other instances, it may be desirable to run the subsequent carbohydrate conversion process outside of the facility in which the processing method of the present invention is performed, e.g. outside of the pulping plant from which the initial composition was drawn, e.g. from the SSL stream of a (bi)sulfite pulping process. Generally, however, it will be advantageous from the standpoint of overall process integration to run any downstream process for conversion of separated and concentrated carbohydrate into compounds chemically distinct from this carbohydrate within the production cycle of an existing industrial process, e.g. within the cycle of a wood or biomass pulping process, and the collecting of the resulting carbohydrate-enriched composition may represent an interim pooling of the carbohydrate-enriched composition within such an integrated cycle, prior to feeding the carbohydrate-enriched composition into subsequent carbohydrate conversion processes, e.g. microbial carbohydrate conversion processes.

As explained hereinabove, the carbohydrate conversion processes to which the carbohydrate-enriched composition may subsequently be subjected may involve or depend on, in whole or in part, microorganisms for the desired chemical conversion. It is well-known that many microorganisms possess the natural, that is endogenous or wild type, ability to convert substrate carbohydrates into further products which are chemically distinct from the substrate carbohydrates. Fermentation is one well-known example. However, as the skilled person is well aware, it is possible to genetically engineer microorganisms to express foreign genes encoding proteins/enzymes which effect chemical conversions of carbohydrates which the wild type microorganisms, that is lacking such foreign genes, would not normally be able to effect. One possible conversion made possible by genetic modification of microorganisms is the conversion of certain carbohydrates into industrially valuable products such as 1,4-butanediol, lactic acid, polylactic acid, one or more butenes, butadiene (1,3-butadiene), acrylic acid, polyhydroxyalkanoates, succinic acid, adipic acid, n-butanol, isobutanol, 1,2-butanediol, 2,3-butanediol, 1,3-propanediol, 1,2-propanediol, isoprene, mannitol, sorbitol, xylitol, glucaric acid, mannaric acid, xylaric acid, gluconic acid, mannonic acid, xylonic acid, and citric acid, to name just a few. Succinic acid, 1,4-butanediol, lactic acid and polylactic acid are especially preferred. The wide range of products which genetic engineering has made accessible, and will continue to make accessible starting from suitable carbohydrate compositions illustrates the great advantage of the methods of the present invention, as these methods give the skilled person the raw materials, in the form of carbohydrate-enriched compositions, needed to take advantage of the vast potential which genetic modification of microorganisms in carbohydrate conversion provides.

In a further aspect, the present invention provides a carbohydrate-enriched composition producible by any of the methods described herein. In a still further aspect, the present invention provides a carbohydrate-enriched composition produced by any of the methods described herein. It is to be generally understood that the carbohydrate-enriched composition comprises at least one carbohydrate which was originally present in the initial composition.

A further aspect of the invention relates to the use of a carbohydrate-enriched composition produced or producible by the method of the invention, or any embodiments thereof as described herein, as a substrate for conversion of said carbohydrate into a compound chemically distinct from said carbohydrate. The used carbohydrate-enriched composition produced or producible by the method of the invention, or any embodiments thereof as described herein, comprises at least one carbohydrate originally present in the initial composition. In certain embodiments, said conversion is effected by a microorganism. In certain embodiments, the compound chemically distinct from said carbohydrate is chosen from the group consisting of 1,4-butanediol, lactic acid, polylactic acid, one or more butenes, butadiene (1,3-butadiene), acrylic acid, polyhydroxyalkanoates, succinic acid, adipic acid, n-butanol, isobutanol, 1,2-butanediol, 2,3-butanediol, 1,3-propanediol, 1,2-propanediol, isoprene, mannitol, sorbitol, xylitol, glucaric acid, mannaric acid, xylaric acid, gluconic acid, mannonic acid, xylonic acid, and citric acid. In an especially preferred embodiment the compound is chemically distinct from said carbohydrate is succinic acid, 1,4-butanediol, lactic acid, or polylactic acid. The skilled person will understand that the above list of compounds chemically distinct from said carbohydrate is not exhaustive. In particular, the skilled person will understand that microorganisms may be engineered to convert carbohydrates to any number of compounds chemically distinct from carbohydrates, and that virtually any conversion of one or more carbohydrates to a compound chemically distinct therefrom may be effected given the availability of suitable genes for incorporation into said microorganisms.

In its most general form, the present invention thus relates to a processing method relating to processing an initial composition comprising a lignosulfonate and a carbohydrate as described above, and resulting in a carbohydrate-enriched composition which is well-suited for feeding into a downstream carbohydrate conversion process, e.g. a downstream microbial carbohydrate conversion process. Which sequence of operations is suitable for the concentration procedure depends on the required feed specifications for the subsequent carbohydrate conversion process and the required feed properties of the compositions to optionally be returned to an ongoing process, e.g. in a pulp mill. The compositions to optionally be returned to an ongoing process include the LCCD composition (fraction 1) obtained from the separation procedure, as well as any compositions obtained (e.g. as an permeate or a condensate) from the concentration procedure. In general, the concentration of mixed carbohydrates and the content of non-carbohydrate compounds in the carbohydrate stream with the purpose to feed a conversion process can be tailored by modifying the process parameters of the unit operations for separation and concentration and by selecting the sequence of unit operations within the concentration procedure.

Accordingly, the method of the present invention may for instance advantageously be carried out in the following sequences:
1) separation procedure + concentration procedure with a semipermeable membrane
2) separation procedure + concentration procedure with a semipermeable membrane + concentration procedure with an evaporation step;
3) separation procedure + concentration procedure with an evaporation step.

As set out hereinabove, other sequences within the concentration procedure are conceivable, as the skilled person will tailor obtaining a final carbohydrate-enriched composition as needed. As also set out hereinabove, in certain embodiments it is advantageous that the concentration procedure minimally comprises a step employing a semipermeable membrane.

In summary, the present invention comprising the described sequence of unit operations (separation procedure + concentration procedure), allows several key advantages over the prior art for integrating a carbohydrate conversion process into an existing production process, e.g. an existing pulp production plant, based on e.g. the (Mg)(bi)sulfite process with an adsorptive (Mg)(bi)sulfite recovery cycle following the concept of an energy- and material-integrated biorefinery. These advantages include:
1) The potential that no additional chemical compounds are introduced into the secondary chemical recovery cycle, e.g. sulfite recovery boilers with SO₂ adsorption stages during operation of a carbohydrate conversion process in a (Mg)(bi)sulfite based pulp mill.
2) Thickening of the carbohydrate stream to significantly higher carbohydrate concentrations in the carbohydrate-enriched composition than in the initial composition. This thickening can be advantageous for optimizing later carbohydrate conversion processes.
3) Tailored purification of the initial composition, because a large share of phenolic inhibitors (e.g. lignosulfonates) are separated from the initial composition, e.g. the SSL stream in the first separation procedure. Surprisingly, potential inhibitors of planned downstream conversion processes and color precursors can be separated much more efficiently after the separation procedure, e.g. a chromatographic separation step, and their final concentration can be tailored. Components that are required for chemical recovery are recovered in high yield from the first separation procedure, from the subsequent concentration procedure or from the first separation procedure and from the subsequent concentration procedure. Convertible carbohydrates not comprising the main carbohydrate component (e.g. xylose from hardwoods, annual plants or dedicated crops or mannose from softwoods) such as glucose, arabinose, galactose, rhamnose and disaccharides surprisingly remain in high yield in the carbohydrate-enriched composition and thus contribute to yields and volumetric yields of downstream conversion processes.
4) Carbohydrate-enriched compositions tailored in concentration and composition by the method of the invention can surprisingly lead to higher yields, rates, and volumetric yields of subsequent conversion processes (as shown in following examples), and to higher product concentrations.
5) Carbohydrate-enriched compositions tailored in concentration and composition by the method of the invention require smaller process equipment volumes for any subsequent carbohydrate conversion process due to shorter residence time requirements and higher educt and product concentrations in the conversion process.
6) Membrane separation processes with the aim of purifying carbohydrates e.g. SSL-derived carbohydrates, can be run in a more stable way due to reduced fouling tendencies. Lignosulfonates are known to sometimes cause membrane fouling. If fouling is prevented by separation of lignosulfonates prior the membrane separation process, higher membrane volumetric flux per membrane area is achievable and lower downtimes are required for membrane cleaning operations.
7) Lignosulfonate-containing fractions from the separation procedure, as well as condensates and permeates from the concentration procedure can be reintegrated into existing operations, e.g. in a pulp mill, for the best-suited purpose and for the maximum possible gain in value.

All publications referenced herein are hereby incorporated by reference.

### Examples

The invention will now be illustrated in greater detail in the following examples. It is to be understood that the following examples merely illustrate, but do not limit the scope of the invention described and claimed herein. Accordingly, the skilled person will be able to recognize deviations from the invention as described and claimed herein without departing from the spirit or scope of the invention.

### Example 1: Expected solute concentrations in SSLs before and after the separation procedure (generation of lignosulfonate-depleted composition from initial composition)

The present example shows results expected when subjecting SSL feeds (initial composition) from pulping of spruce wood and beech wood to a chromatographic separation procedure. Table 1, below, shows typical concentrations of relevant solutes in an SSL feed, both as expected concentrations as well as an expected concentration range in terms of total carbohydrates (monosaccharide), lignosulfonate, total sulfur, magnesium ion, acetic acid, furfural, 5-HMF and water. The indicated concentrations represent typical concentrations in the SSL feed itself, that is before chromatographic separation is performed.

**Table 1. Expected composition of SSL from a spruce wood and beech wood sulfite cook before separation and concentration**

| | | | | |
|---|---|---|---|---|
| | **SSL Spruce** | | **SSL Beech** | |

| Component | Expected conc. [g/L] | Expected Conc. Range [g/l] | Expected conc. [g/L] | Expected Conc. Range [g/l] |
|---|---|---|---|---|
| Total Monosaccharide s | 150 | 100 - 200 | 180 | 130 - 230 |
| Glucose | 27 | 20 - 35 | 18 | 12 - 24 |
| Xylose | 28 | 20 - 35 | 137 | 100 - 180 |
| Mannose | 81 | 60 - 100 | 12 | 6 - 14 |
| Arabinose | 4 | 2 - 6 | 2 | 1 - 4 |
| Galactose | 10 | 6 - 14 | 7 | 4 - 10 |
| Rhamnose | 1 | 0 - 2 | 4 | 0 - 7 |
| Lignosulfonate | 450 | 350 - 550 | 450 | 350 - 550 |
| Total Sulfur | 65 | 40 - 80 | 65 | 40 - 80 |
| Mg | 38 | 30 - 50 | 39 | 30 - 50 |
| Acetic acid | 6 | 4 - 8 | 18 | 10 - 25 |
| Furfural | traces | traces | 0.06 | 0.02 - 0.20 |
| 5-HMF | 0.7 | 0.3 - 1.0 | 0.3 | 0.1 - 0.5 |
| Water | 550 | 450 - 600 | 520 | 450 - 600 |

Chromatographic separation is performed on each of the SSL feeds (initial compositions), from spruce and beech respectively, indicated above.

Table 2, below, shows expected concentrations of selected relevant solutes in the lignosulfonate-depleted composition (2^{nd} chromatographic fraction ("F2")) from the chromatographic separation described above.

**Table 2. Expected concentration of Fraction 2 (F2, Carbohydrate fraction) after chromatography**

| | F2 - Spruce | F2-Beech |
|---|---|---|
| Component | Expected conc. [g/L] | Expected conc. [g/L] |
| Total Monosaccharides | 140 | 169 |
| Acetic acid | 6 | 17 |
| Furfural | traces | 0.1 |
| 5-HMF | 0.6 | 0.3 |
| Water | 895 | 862 |

As can clearly be seen from the comparison of Tables 1 and 2, the total amount of water in the lignosulfonate-depleted composition following chromatographic separation is expected to increase drastically, while the carbohydrate (monosaccharide) concentration is expected to remain approximately the same. This is due to a replacement of such materials as lignosulfonate with water, with the ultimate effect of reducing the lignosulfonate concentration. That is, the water: carbohydrate (monosaccharide) ratio is markedly increased while maintaining the feed carbohydrate (monosaccharide) concentration in the SSL largely unchanged.

### Example 2: Expected increase in carbohydrate concentration in the 2^{nd} fraction from chromatographic separation, by a subsequent concentration (generation of carbohydrate (monosaccharide)-enriched composition from lignosulfonate-depleted composition)

Lignosulfonate-depleted compositions (2^{nd} fraction of a chromatographic separation procedure (see Table 2)) are subjected separately to nanofiltration (NF) and diafiltration (DF) concentration. Table 3 shows expected results with respect to certain relevant solutes originally present in the SSL (initial composition).

**Table 3. Expected concentration ranges of Retentate Fraction (Carbohydrate (monosaccharide) fraction) after membrane separation processes nanofiltration (NF) and diafiltration (DF) employing a nanofiltration membrane of the type Oltremare NANO9 at 60°C and 50 bar pressure**

| | **Retentate NF - Spruce** | **Retentate DF - Spruce** | **Retentate NF - Beech** | **Retentate DF - Beech** |
|---|---|---|---|---|
| Component | Expected conc. [g/L] | Expected conc. [g/L] | Expected conc. [g/L] | Expected conc. [g/L] |
| Total Monosaccharides | 200 - 400 | 200-400 | 250 - 400 | 250 - 400 |
| Acetic acid | 3-7 | traces - 7 | 10 - 16 | traces - 16 |
| Furfural | Traces | traces | 0.02 - 0.05 | traces - 0.05 |
| HMF | 0.4 - 0.9 | traces - 0.9 | 0.2 - 0.4 | traces - 0.4 |

Table 3 illustrates that carbohydrates (monosaccharide) can be selectively concentrated in the retentate by nanofiltration while decreasing the concentration of potential inhibiting impurities (i.e. acetic acid, furfural, 5-HMF). The expected water:carbohydrate (monosaccharide) ratio is compared to that of fraction 2 from the chromatographic separation procedure (shown in Table 2; corresponds to "lignosulfonate-depleted composition"). This ratio can be further reduced and adjusted as needed/desired by means of further diafiltration.

Further, the lignosulfonate-depleted compositions (2^{nd} fraction of a chromatographic separation procedure (Table 2)) are subjected separately to a concentration by evaporation. Table 4 shows expected results with respect to certain relevant solutes originally present in the SSL (initial composition).

**Table 4. Expected concentration ranges of Evaporate Fraction (carbohydrate (monosaccharide) fraction) after evaporation**

| | **Evaporate - Spruce** | **Evaporate - Beech** |
|---|---|---|
| Component | Expected concentration [g/L] | Expected concentration [g/L] |
| Total Monosaccharides | 200 - 600 | 250 - 700 |
| Acetic acid | 6 - 10 | 18 - 30 |
| Furfural | Traces | 0.01 - 0.1 |
| 5-HMF | 0.8 - 4.5 | 0.3 - 2.0 |

Table 4 illustrates that carbohydrates (monosaccharide) can be selectively concentrated in the retentate by evaporation while decreasing the inhibitor (i.e. acetic acid, furfural) : carbohydrate ratio compared to Fraction 2 (Table 2). 5-HMF is not separated from carbohydrates (monosaccharide) during evaporation.

### Example 3: Preparation of a carbohydrate (monosaccharide) composition with a total carbohydrate concentration near 600 g/L and an HMF concentration < 0.8 ail from spruce SSL

The aim is to produce a carbohydrate (monosaccharide) enriched composition as a feed for a fed-batch fermentation, during which the carbohydrates are converted to a water-miscible fermentation product with a boiling point above that of water, e.g. 1,4-butanediol. The production strain in this fermentation is sensitive to 5-HMF and furfural. In the presence of these compounds and depending on their concentration, growth of the microorganisms is inhibited or production rates are significantly lower than in these compounds' absence. To achieve the desired total carbohydrate concentration in the carbohydrate composition, Fraction 2 after chromatography is further concentrated and purified by means of nanofiltration employing a nanofiltration membrane Oltremare NANO9 at 50 bar pressure and 60°C. The nanofiltration process is performed until the total carbohydrate composition in the retentate increases to approximately 300 g/L. Then the mode of the membrane separation process is switched to diafiltration and water is added to the feed. The diafiltration is run until the 5-HMF concentration in the retentate was below 0.4 g/L. After diafiltration, the retentate is concentrated by vacuum evaporation until the total carbohydrate concentration exceeds 600 g/L. The expected yield of total carbohydrates in this example is 83%. Samples may be taken before, during and after the procedure to check the concentration of total carbohydrates (monosaccharides, determined by HPLC), 5-HMF (determined with GC-FID) and acetic acid (determined with ion chromatography). Furfural concentrations are not monitored during the experiment, as the initial concentration in spruce SSL is very low. Table 5 shows the concentrations of total carbohydrates (monosaccharide), acetic acid, and furfural during this sequence of processes in the order of the process steps.

**Table 5. Expected concentration ranges of Retentate Fraction (carbohydrate fraction) after the membrane separation processes nanofiltration (NF) and diafiltration (DF) employing membrane Oltremare NANO9 at 60°C and 50 bar pressure for nanofiltration and diafiltration.**

| | **Starting point: Fraction 2 after chromatography** | **Retentate after nanofiltration** | **Retentate after diafiltration** | **Concentrate after evaporation** |
|---|---|---|---|---|
| Component | Expected concentration [g/L] | Expected concentration [g/L] | Expected concentration [g/L] | Expected concentration [g/L] |
| Total Monosaccharides | 140 | 303 | 272 | 603 |
| Acetic acid | 6.0 | 2.4 | 0.9 | 1.4 |
| Furfural | traces | Not calculated | Not calculated | Not calculated |
| HMF | 0.64 | 0.71 | 0.36 | 0.73 |

As a reference, fraction 2 after chromatography is vacuum evaporated until the concentration of total carbohydrates exceeds 600 g/L. Samples are taken before, during and after the procedure to check the concentration of total carbohydrates, 5-HMF and acetic acid (determinable according to NREL Technical Report: NREL/TP-510-42623 (2008)).

Expected concentrations of substances in this concentrate after evaporation alone are as follows: 611 g/L total carbohydrates, 9.8 g/L acetic acid, and 2.71 g/L 5-HMF. Furfural concentration is not calculated.

It is evident from this example that application of a combination of nanofiltration (concentration procedure employing a semipermeable membrane), diafiltration (concentration procedure employing a semipermeable membrane), and evaporation (concentration procedure employing an evaporation step) to concentrate fraction 2 after chromatography (separation procedure) may be advantageous in achieving the target of ∼ 600 g/L total carbohydrates while at the same time reducing 5-HMF concentrations to below 0.8 g/L. With evaporation alone following chromatographic separation, 600 g/L carbohydrate concentration can be achieved, but at the same time the 5-HMF concentration remains above the level required for a fed batch fermentation employing a production strain sensitive to 5-HMF and furfural.

The present example illustrates how the skilled person may combine various separation and concentration procedures, possibly in an iterative fashion, to arrive at a final carbohydrate-enriched composition which satisfies the particular requirements or constraints of intended downstream processes.

### Example 4: Fermentation to ethanol with Saccharomyces cerevisiae

This example illustrates the expected efficiency/yield of fermentation to ethanol performed on various intermediate results of the present invention. In each case, *Saccharomyces cerevisiae* are employed to ferment carbohydrates from a spruce SSL stream into ethanol, and the amount of ethanol resulting from this fermentation is an indicator of fermentation efficiency. In Fermentation 1, spruce SSL not subjected to the method of the present invention is fermented directly (the substrate of Fermentation 1 corresponded to the "initial composition" of the present invention). In Fermentation 2, fermentation is performed on a sample of spruce SSL subjected to only the (chromatographic) separation procedure of the present invention (i.e. the substrate of Fermentation 2 corresponds to the "lignosulfonate-depleted composition" of the present invention). In Fermentation 3, fermentation is performed on a sample of spruce SSL subjected to both the (chromatographic) separation procedure and the (nanofiltration) concentration procedure of the present invention (i.e. the substrate of Fermentation 3 corresponds to the "carbohydrate-enriched composition" of the present invention). The fermentations are carried out in shake flask cultures for 96 hours at 30°C. The pH of the substrate is adjusted to 4.0 with NaOH. The amount of inoculum is 5% of the total volume. Final ethanol concentration is measured with GC-FID.

Table 6 illustrates total expected carbohydrate and inhibitor concentrations prior to the respective fermentations to ethanol, as well as the expected ethanol yield (g ethanol / kg carbohydrate) and concentration following the respective fermentations.

**Table 6: Expected substrate composition (total carbohydrate (monosaccharide), inhibitors) and product yields and expected concentrations after ethanol fermentations.**

| | Fermentation 1 | Fermentation 2 | Fermentation 3 |
|---|---|---|---|
| Total Monosaccharides [g/l] | 162 | 155 | 238 |
| Acetic acid [g/l] | 6.3 | 6.2 | 3.7 |
| 5-HMF [g/l] | 0.53 | 0.54 | 0.32 |
| | | | |
| Ethanol yield [g/kg total monosaccharides] | 0 | 311 | 352 |
| Ethanol concentration [g/L] | 0 | 48 | 83 |

As illustrated in Table 6 above, fermentation is completely inhibited by spruce SSL, while both the expected yield and concentration of ethanol are highest in Fermentation 3, when the substrate (carbohydrate-enriched composition) is prepared according to the invention employing a sequence comprising separation (e.g. chromatography) and concentration (e.g. nanofiltration). This illustrates not only how the retention of inhibiting substances in the carbohydrate-enriched composition may have a deleterious effect on subsequent microbial conversion, but also how the method of the invention can successfully remove such inhibiting substances while increasing the initial concentration of carbohydrate (monosaccharide) substrate so as to increase desired product yield due to microbial conversion.

### Example 5: Fermentation to lactic acid with Lactobacillus sp.

Similar to Example 4, above, this example illustrates the expected efficiency/yield of fermentation to lactic acid performed on various intermediate results of the present invention. In each case, *Lactobacillus sp.* are employed to ferment carbohydrates from a spruce SSL stream into lactic acid, and the amount of lactic acid resulting from this fermentation is an indicator of fermentation efficiency. In Fermentation 4, spruce SSL not subjected to the method of the present invention is fermented directly (the substrate of Fermentation 4 corresponds to the "initial composition" of the present invention). In Fermentation 5, fermentation is performed on a sample of spruce SSL subjected to only the (chromatographic) separation procedure of the present invention (i.e. the substrate of Fermentation 5 corresponds to the "lignosulfonate-depleted composition" of the present invention). In Fermentation 6, fermentation is performed on a sample of spruce SSL subjected to both the (chromatographic) separation procedure and the (diafiltration) concentration procedure of the present invention (i.e. the substrate of Fermentation 6 corresponded to the "carbohydrate-enriched composition" of the present invention). The fermentations are carried out in stirred bioreactors for 72 hours at 45°C. The pH is adjusted to pH 6.0 and controlled with NaOH. The amount of inoculum is 10% of the total volume. Final lactate concentration is determined enzymatically using D/L-lactic acid kits from r-biopharm (www.r-biopharm.com).

Table 7 illustrates total expected carbohydrate and inhibitor concentrations prior to the respective fermentations to lactic acid, as well as the expected lactic acid yield (g lactic acid / kg carbohydrate) and concentration following the respective fermentations.

**Table 7. Expected substrate composition (total carbohydrate (monosaccharide), inhibitors) and expected product yields and concentrations after lactic acid fermentations.**

| | Fermentation 4 | Fermentation 5 | Fermentation 6 |
|---|---|---|---|
| Total Monosaccharides [g/l] | 180 | 169 | 281 |
| Acetic acid [g/l] | 6.3 | 6.2 | 1.0 |
| 5-HMF [g/l] | 0.53 | 0.54 | 0.09 |
| Lactic acid yield [g/kg total monosaccharides] | 0 | 489 | 730 |
| Lactic acid concentration [g/l] | 0 | 83 | 205 |

As illustrated in Table 7 above, fermentation is completely inhibited by spruce SSL, while both the expected yield and concentration of lactic acid are highest in Fermentation 6, when the substrate (carbohydrate-enriched composition) is prepared according to the invention employing a sequence comprising separation (e.g. chromatography) and concentration (e.g. diafiltration). This illustrates not only how the retention of inhibiting substances in the carbohydrate-enriched composition may have a deleterious effect on subsequent microbial conversion, but also how the method of the invention can successfully remove such inhibiting substances while increasing the initial concentration of carbohydrate (monosaccharide) substrate so as to increase desired product yield due to microbial conversion.

## Claims

1. A method of processing an initial composition comprising a lignosulfonate and a carbohydrate, said method comprising:
• subjecting the initial composition to a separation procedure whereby an amount of the lignosulfonate is removed from the initial composition, thereby yielding a **l**ignosulfonate-**c**ontaining and **c**arbohydrate-**d**epleted (LCCD) composition and a lignosulfonate-depleted composition; and
• subjecting the lignosulfonate-depleted composition to a concentration procedure whereby the carbohydrate concentration is increased relative to the carbohydrate concentration in the initial composition, relative to the carbohydrate concentration in the lignosulfonate-depleted composition or relative to the carbohydrate concentration in both the initial composition and the lignosulfonate-depleted composition, thereby yielding a carbohydrate-enriched composition.

2. The method of claim 1, wherein the carbohydrate in the carbohydrate-enriched composition is not crystallized.

3. The method of claim 1 or 2, wherein the separation procedure is a chromatographic separation procedure.

4. The method of any of the preceding claims, wherein the concentration procedure employs
• a semipermeable membrane;
• an evaporation step; or
• a semipermeable membrane and an evaporation step.

5. The method of claim 4, wherein the concentration procedure employs a semipermeable membrane and the molecular weight cutoff of the semipermeable membrane is chosen so as to prevent the passage of at least one carbohydrate through the membrane.

6. The method of any of the preceding claims, wherein the initial composition additionally comprises metal ions such as Mg²⁺, Ca²⁺, Na⁺ or any combination thereof, sulfates, sulfites, furfural, 5-hydroxymethyl furfural, acetic acid, formic acid, one or more aldonic acid or any combination thereof.

7. The method of any of the preceding claims, wherein the carbohydrate comprises xylose, mannose, glucose, galactose, arabinose, rhamnose ribose, fucose or any combination thereof; disaccharides, oligosaccharides or polysaccharides of any thereof or combinations thereof; hemicelluloses; hydrolysis products of hemicelluloses; or any combination thereof.

8. The method of any of the preceding claims, wherein the initial composition is a composition resulting from pulping or pretreating a lignocellulosic material, such as wood, preferably using H₂SO₃, solutions of SO₂, or both, preferably wherein the initial composition is a spent sulfite liquor.

9. The method of any of the preceding claims, wherein the initial composition comprising the lignosulfonate and the carbohydrate is obtained from the stream of a concurrently ongoing procedure.

10. The method of claim 9, wherein the concurrently ongoing procedure is a concurrently ongoing pulping procedure, preferably a wood or lignocellulosic biomass pulping procedure.

11. The method of claim 9 or 10, further comprising the step of returning the LCCD-composition to the stream of the concurrently ongoing procedure.

12. The method of any of claims 9 or 10, as dependent from any of claims 4-8, further comprising the step of returning the permeate of the concentration procedure employing a semipermeable membrane, the condensate of the concentration procedure employing an evaporation step or said permeate and said condensate to the stream of the concurrently ongoing procedure.

13. A method of preparing a carbohydrate-enriched composition, comprising the steps of:
• performing the method of any of the preceding claims; and
• collecting the resulting carbohydrate-enriched composition.

14. The method of any of the preceding claims, further comprising converting a carbohydrate or carbohydrates in the carbohydrate-enriched composition to a compound chemically distinct from said carbohydrate or carbohydrates.

15. A carbohydrate-enriched composition produced or producible by the method of any of claims 1-13.

16. Use of a carbohydrate-enriched composition of claim 15, or produced or producible by the method of any of claims 1-13 as a substrate for conversion of said carbohydrate into a compound chemically distinct from said carbohydrate.
